(19) Europäisches Patentamt / European Patent Office / Office européen des brevets

(11) **EP 4 527 836 A1**

(12) **EUROPEAN PATENT APPLICATION**
published in accordance with Art. 153(4) EPC


(43) Date of publication:
**26.03.2025 Bulletin 2025/13**

(21) Application number: **23826380.0**

(22) Date of filing: **20.06.2023**

(51) International Patent Classification (IPC):
***C07D 401/14*** (2006.01) ***A61P 1/00*** (2006.01)
***A61P 37/08*** (2006.01) ***A61P 9/04*** (2006.01)
***A61P 13/12*** (2006.01) ***A61P 1/16*** (2006.01)

(52) Cooperative Patent Classification (CPC):
**A61P 1/00; A61P 1/16; A61P 9/04; A61P 13/12; A61P 37/08; C07D 401/14**

(86) International application number:
**PCT/CN2023/101233**

(87) International publication number:
**WO 2023/246732 (28.12.2023 Gazette 2023/52)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.06.2022 CN 202210701699**



(71) Applicant: **SHANGHAI JEYOU PHARMACEUTICAL CO., LTD.**
**Shanghai 201315 (CN)**

(72) Inventors:
- **ZHANG, Jin**
**Shanghai 201315 (CN)**
- **HUANG, Jian**
**Shanghai 201315 (CN)**

(74) Representative: **Gille Hrabal Partnerschaftsgesellschaft mbB Patentanwälte Brucknerstraße 20 40593 Düsseldorf (DE)**


(54) **BENZOHETEROCYCLIC SUBSTITUTED TETRAHYDROISOQUINOLINE COMPOUND SALT FORM AND PREPARATION METHOD THEREFOR**


(57) Disclosed are a benzoheterocyclic substituted tetrahydroisoquinoline compound salt form and a preparation method therefor. Specifically, disclosed are an amorphous form and salt form of the compound as shown in formula (I), and a use thereof to inhibit NHE-mediated sodium ion or hydrogen ion reverse transfer.


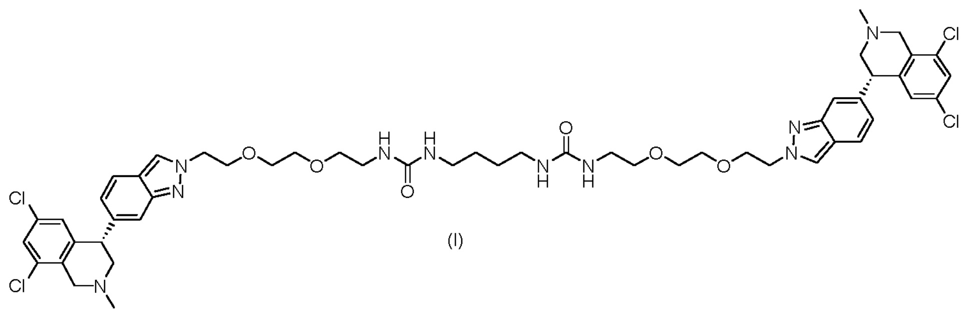

(I)

EP 4 527 836 A1

## Description

The present application claims priority of CN202210701699.9 filed on June 20, 2022.

## TECHNICAL FIELD

The present disclosure relates to the field of medicinal chemistry, and particularly to a salt form of a benzoheterocyclic ring-substituted tetrahydroisoquinoline compound and a preparation method therefor.

## BACKGROUND

Phosphate is an important mineral that regulates many metabolic processes, such as signal transduction, energy production, mineral metabolism, etc., and is absorbed mainly in the small intestine, filtered by the kidneys, and then reabsorbed or excreted through the renal tubules. Thus, despite differences in daily phosphate intake, serum phosphate concentrations remain within a physiological range. In patients with advanced chronic kidney disease (CKD), hyperphosphatemia occurs due to a substantial loss of the function of the kidney to metabolize phosphorus. Studies have shown that hyperphosphatemia is associated with multiple adverse clinical outcomes in patients with CKD, including: induction of vascular calcification, increased incidence and death risk of cardiovascular diseases, secondary hyperparathyroidism, metabolic bone disease or ectopic calcification resulting from renal osteodystrophy, promotion of renal failure and the progression of cardiovascular diseases.

Currently, the main treatment measures for hyperphosphatemia are a low-phosphate diet, hemodialysis treatment, and administration of a phosphate binding agent with meals. Clinical experience has shown that it is relatively difficult to control phosphate intake through diet; hemodialysis has a limited efficiency; and therefore, the use of the phosphate binding agent is an important treatment method for lowering serum phosphorus level at present. Currently, the phosphate binding agent commonly used in clinics mainly includes two types: a phosphate binding agent containing a metal ion (calcium/magnesium/iron/lanthanum) and an ion exchange resin-type binding agent (sevelamer or sevelamer carbonate). For the phosphate binding agent containing the metal ion, patients need to strengthen the management of metal ions in the drug, and the phosphorus binding effect of the drug is relatively poor due to the influence of pH, thereby tending to cause diarrhea and intolerance in patients. The latter binds to phosphorus through ion exchange, is not absorbed by the gastrointestinal tract, reduces accumulation, and has fewer side effects than the former. However, these two types of drugs have high dosages, high prices, and poor patient compliance.

Currently, two main modes for the absorption of phosphates in the intestinal tract are known: passive cellular bypass transport and active transport dependent on transport proteins, and passive cellular bypass phosphate transport is considered to be the main reason for the absorption of phosphates in humans. The cellular bypass phosphate transport is mainly driven by the concentration gradient of phosphates, which are absorbed by a tight junction complex formed between cells. It has been shown in the literature that this tight junction complex has permeability specificity for specific ions through the regulation of signal transduction. Sodium-hydrogen antiporter 3 (NHE3/SLC9A3) is a gastrointestinal transport protein expressed on the apical surface of intestinal epithelial cells and mainly responsible for maintaining the balance of sodium ions, which can affect sodium absorption in the intestinal tract by inhibiting the activity of NHE3 in the intestinal tract, thereby changing the concentration of hydrogen ions in the intestinal epithelial cells and further affecting the change of local pH; and lower the permeability of the tight junction complex formed between cells to phosphates, thereby reducing the absorption of phosphates through the cellular bypass. In clinical practice, the need for serum phosphorus control in patients with advanced CKD has not been met yet, and thus, it is necessary to further develop drugs for lowering serum phosphorus with different mechanisms.

The application with the application No. PCT/CN2021/139314 (filed on December 17, 2021) provides a compound that inhibits NHE-mediated antiport of sodium or hydrogen ions, which has a structure as shown below:

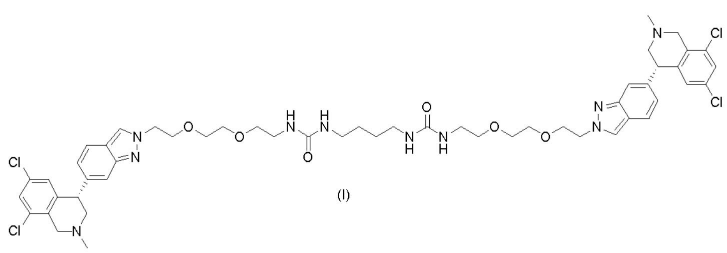

(I)

.

## SUMMARY

In one aspect of the present disclosure, the present disclosure discloses an amorphous form of a compound represented by formula (I),

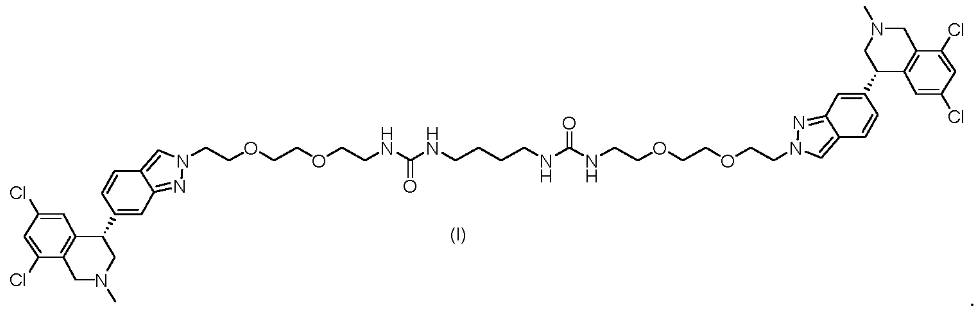

(I)

In some embodiments of the present disclosure, the amorphous form described above has an XPRD pattern substantially as shown in FIG. 1.

In another aspect of the present disclosure, the present disclosure further provides 1,5-naphthalenedisulfonate of a compound represented by formula (I), which has a structure as shown below:

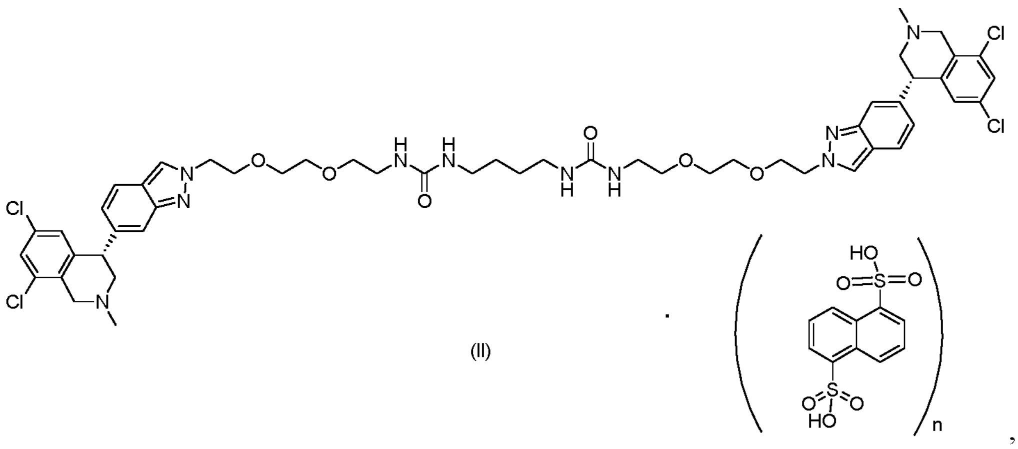

(II)

wherein n = 0.9-2.0.

In another aspect of the present disclosure, the present disclosure further provides an amorphous form of a 1,5-naphthalenedisulfonate of a compound represented by formula (I), wherein the molar ratio of the compound represented by formula (I) to 1,5-naphthalenedisulfonic acid is 1.0:(0.9-2.0).

In some embodiments of the present disclosure, the molar ratio of the compound represented by formula (I) to 1,5-naphthalenedisulfonic acid is 1.0:0.9, 1.0:1.0, 1.0:1.2, 1.0:1.5, 1.0:1.8, or 1.0:2.0.

In some embodiments of the present disclosure, the amorphous form of the 1,5-naphthalenedisulfonate described above has an XPRD pattern substantially as shown in FIG. 2 or FIG. 4 or FIG. 7.

In some embodiments of the present disclosure, the amorphous form of the 1,5-naphthalenedisulfonate described above has a TGA/mDSC substantially as shown in FIG. 5 or FIG. 8.

In some embodiments of the present disclosure, the amorphous form of the 1,5-naphthalenedisulfonate described above has a $^1$H NMR spectrum substantially as shown in FIG. 6 or FIG. 9 or FIG. 10.

In another aspect of the present disclosure, the present disclosure further provides a method for preparing 1,5-naphthalenedisulfonate of a compound represented by formula (I). The method comprises a reaction as shown below,

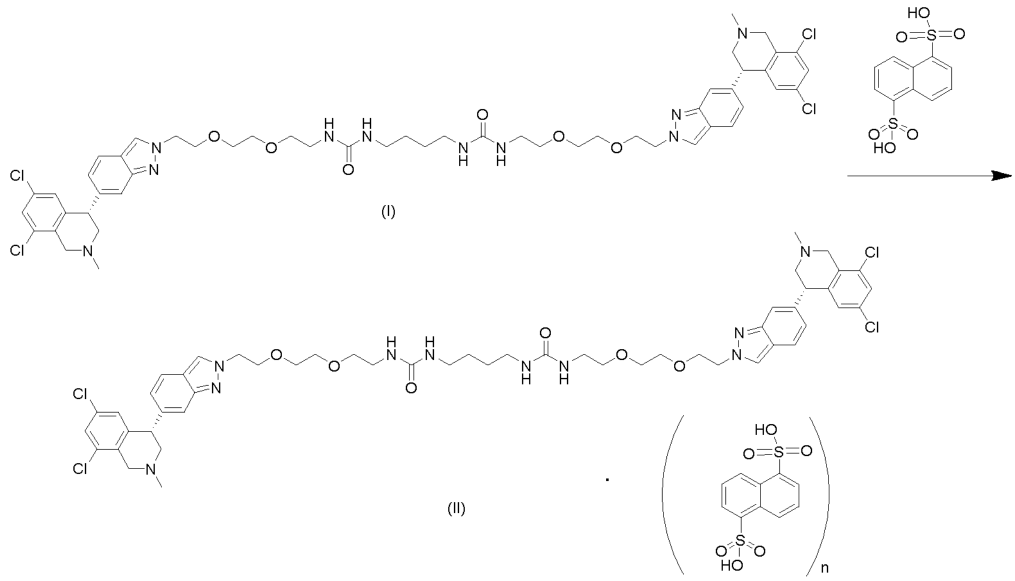

(I)

(II)

wherein n = 0.9-2.0, and a reaction solvent is selected from isopropanol, ethyl acetate, or a mixture of isopropanol and ethyl acetate.

In some embodiments of the present disclosure, the method described above may further comprise at least one of the following additional technical features.

In some embodiments of the present disclosure, n is selected from 0.9, 1.0, 1.1, 1.2, 1.3, 1.4, 1.5, 1.6, 1.7, 1.8, 1.9, and 2.0.

In some embodiments of the present disclosure, the feeding molar ratio of the compound represented by formula (I) described above to 1,5-naphthalenedisulfonic acid is 1:(1-4).

In some embodiments of the present disclosure, the feeding molar ratio of the compound represented by formula (I) described above to 1,5-naphthalenedisulfonic acid is 1:1.0, or 1:1.2, or 1:1.5, or 1:1.8, or 1:2.0, or 1:2.2, or 1:2.5, or 1:2.8, or 1:3.0, or 1:3.2, or 1:3.5, or 1:3.8, or 1:4.0.

In some embodiments of the present disclosure, the molar volume ratio of the compound represented by formula (I) described above to isopropanol is (5-15) mmol:(50-150) mL.

In some embodiments of the present disclosure, the molar volume ratio of the compound represented by formula (I) described above to isopropanol is 5 mmol:50 mL, or 6 mmol:100 mL, or 7 mmol:100 mL, or 8 mmol:100 mL, or 9 mmol:100 mL, or 9.3 mmol:100 mL, or 10 mmol:100 mL, or 11 mmol:100 mL, or 12 mmol:100 mL, or 13 mmol:100 mL, or 14 mmol:150 mL, or 15 mmol:150 mL.

In some embodiments of the present disclosure, the molar volume ratio of the compound represented by formula (I) described above to ethyl acetate is (5-15) mmol:(50-150) mL.

In some embodiments of the present disclosure, the molar volume ratio of the compound represented by formula (I) described above to ethyl acetate is 5 mmol:50 mL, or 6 mmol:100 mL, or 7 mmol:100 mL, or 8 mmol:100 mL, or 9 mmol:100 mL, or 9.3 mmol:100 mL, or 10 mmol:100 mL, or 11 mmol:100 mL, or 12 mmol:100 mL, or 13 mmol:100 mL, or 14 mmol:150 mL, or 15 mmol:150 mL.

In some embodiments of the present disclosure, the method further comprises a stirring treatment, a suction filtration treatment, and a drying treatment after the reaction described above is completed.

In some embodiments of the present disclosure, the stirring treatment described above is performed at room temperature.

In some embodiments of the present disclosure, the stirring treatment described above is performed at room temperature for two days.

In some embodiments of the present disclosure, the suction filtration treatment described above is performed under nitrogen atmosphere.

In some embodiments of the present disclosure, the drying treatment described above is performed under a vacuum condition at room temperature for 2 h.

In another aspect of the present disclosure, the present disclosure further provides a method for preparing an amorphous form of a 1,5-naphthalenedisulfonate of a compound represented by formula (I), wherein the method comprises performing a stirring treatment, a suction filtration treatment, and a drying treatment on the compound represented by formula (I) and 1,5-naphthalenedisulfonic acid in isopropanol or ethyl acetate to obtain the amorphous form of the 1,5-naphthalenedisulfonate of the compound represented by formula (I).

In some embodiments of the present disclosure, the method described above may further comprise at least one of the following additional technical features.

In some embodiments of the present disclosure, the feeding molar ratio of the compound represented by formula (I) described above to 1,5-naphthalenedisulfonic acid is 1:(1-4).

In some embodiments of the present disclosure, the feeding molar ratio of the compound represented by formula (I) described above to 1,5-naphthalenedisulfonic acid is 1:1.0, or 1:1.2, or 1:1.5, or 1:1.8, or 1:2.0, or 1:2.2, or 1:2.5, or 1:2.8, or 1:3.0, or 1:3.2, or 1:3.5, or 1:3.8, or 1:4.0.

In some embodiments of the present disclosure, the molar volume ratio of the compound represented by formula (I) described above to isopropanol is (5-15) mmoL:(50-150) mL.

In some embodiments of the present disclosure, the molar volume ratio of the compound represented by formula (I) described above to isopropanol is 5 mmoL:50 mL, or 6 mmoL:100 mL, or 7 mmoL:100 mL, or 8 mmoL:100 mL, or 9 mmoL:100 mL, or 9.3 mmoL:100 mL, or 10 mmoL:100 mL, or 11 mmoL:100 mL, or 12 mmoL:100 mL, or 13 mmoL:100 mL, or 14 mmoL:150 mL, or 15 mmoL:150 mL.

In some embodiments of the present disclosure, the molar volume ratio of the compound represented by formula (I) described above to ethyl acetate is (5-15) mmoL:(50-150) mL.

In some embodiments of the present disclosure, the molar volume ratio of the compound represented by formula (I) described above to ethyl acetate is 5 mmoL:50 mL, or 6 mmoL:100 mL, or 7 mmoL:100 mL, or 8 mmoL:100 mL, or 9 mmoL:100 mL, or 9.3 mmoL:100 mL, or 10 mmoL:100 mL, or 11 mmoL:100 mL, or 12 mmoL:100 mL, or 13 mmoL:100 mL, or 14 mmoL:150 mL, or 15 mmoL:150 mL.

In some embodiments of the present disclosure, the stirring treatment described above is performed at room temperature.

In some embodiments of the present disclosure, the stirring treatment described above is performed at room temperature for two days.

In some embodiments of the present disclosure, the suction filtration treatment described above is performed under nitrogen atmosphere.

In some embodiments of the present disclosure, the drying treatment described above is performed under a vacuum condition at room temperature for 2 h.

In another aspect of the present disclosure, the present disclosure further provides a hydrochloride of a compound represented by formula (I), which has a structure as shown below:

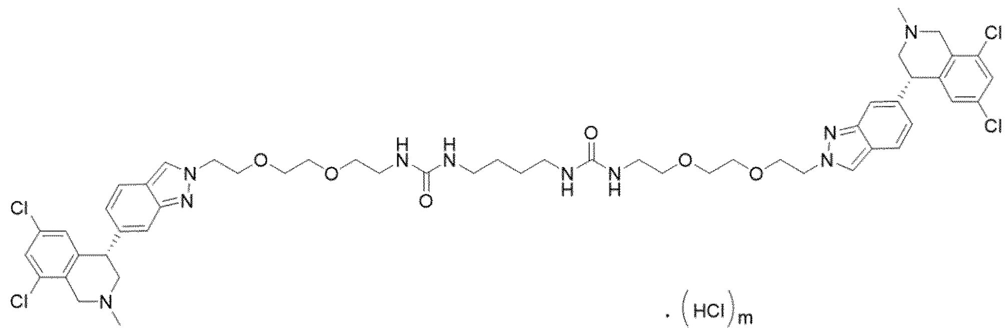

(III)

,

wherein m = 1.6-4.2.

In some embodiments of the present disclosure, m described above is selected from 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, and 4.2.

In some embodiments of the present disclosure, the hydrochloride described above is selected from a monohydrochloride, a dihydrochloride, a trihydrochloride, and a tetrahydrochloride. The inventors have found that the tetrahydrochloride has a higher stability and is less prone to be oxidated than other salt forms.

In another aspect of the present disclosure, the present disclosure further provides a method for preparing a compound represented by formula (III-1). The method comprises a reaction as shown below,

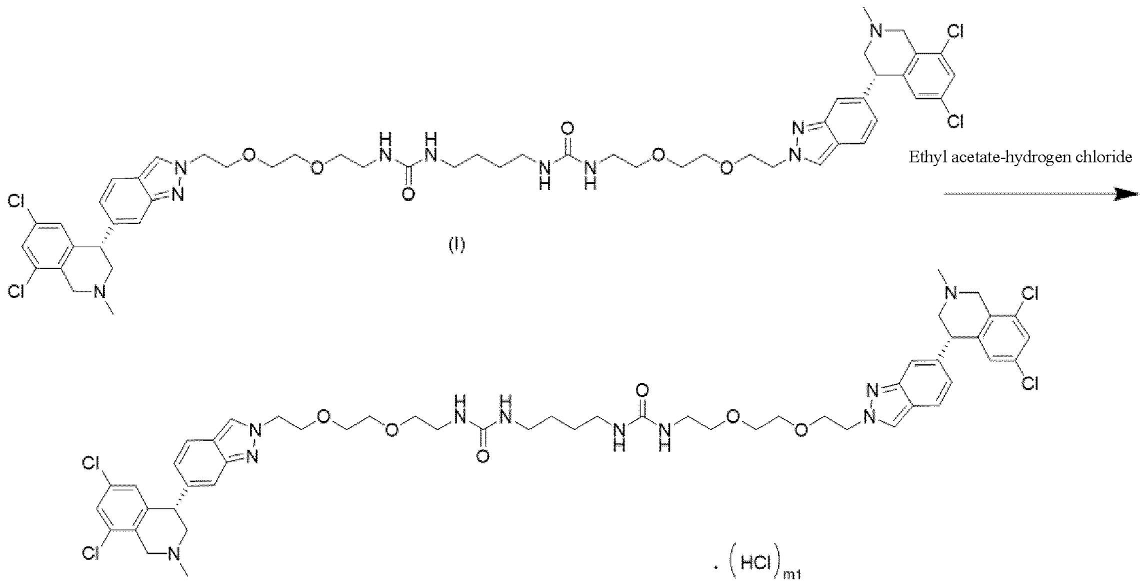


(I)

(III-1)

,

wherein m1 = 1.6-2.5, and a reaction solvent is methyl *tert-butyl* ether.

**In** some embodiments of the present disclosure, the method described above may further comprise at least one of the following additional technical features.

In some embodiments of the present disclosure, the feeding molar ratio of the compound represented by formula (I) described above to ethyl acetate-hydrogen chloride is (2-10):(1-5).

In some embodiments of the present disclosure, the feeding molar ratio of the compound represented by formula (I) described above to ethyl acetate-hydrogen chloride is 2:1, or 3:1, or 4:1, or 5:1, or 6:1, or 7:1, or 8:1, or 9:1, or 10:1, or 3:2, or 4:3, or 5:3, or 5:2, or 5:4, or 6:5, or 7:2, or 7:3, or 7:4, or 7:5, or 7:6, or 8:3, or 8:5, or 8:7, or 9:2, or 9:4, or 9:5, or 9:7, or 9:8, or 10:3, or 10:7, or 10:9.

In some embodiments of the present disclosure, the molar volume ratio of the compound represented by formula (I) described above to methyl *tert*-butyl ether is (5-15) mmol:(50-150) mL.

In some embodiments of the present disclosure, the molar volume ratio of the compound represented by formula (I) described above to methyl *tert*-butyl ether is 5 mmol:50 mL, or 6 mmol:100 mL, or 7 mmol:100 mL, or 8 mmol:100 mL, or 9 mmol: 100 mL, or 9.3 mmol: 100 mL, or 10 mmol: 100 mL, or 11 mmol: 100 mL, or 12 mmol: 100 mL, or 13 mmol: 100 mL, or 14 mmol:150 mL, or 15 mmol:150 mL.

In some embodiments of the present disclosure, the method further comprises a stirring treatment, a suction filtration treatment, and a drying treatment after the reaction described above is completed.

In some embodiments of the present disclosure, the stirring treatment described above is performed at room temperature.

In some embodiments of the present disclosure, the stirring treatment described above is performed at room temperature for two days.

In some embodiments of the present disclosure, the suction filtration treatment described above is performed under nitrogen atmosphere.

In some embodiments of the present disclosure, the drying treatment described above is performed under a vacuum condition at room temperature for 2 h.

In some embodiments of the present disclosure, the molar ratio of the compound represented by formula (I) described above to hydrochloric acid is 1.0:(1.6-2.1).

In another aspect of the present disclosure, the present disclosure further provides a method for preparing a compound represented by formula (III-2) according to the following reaction,

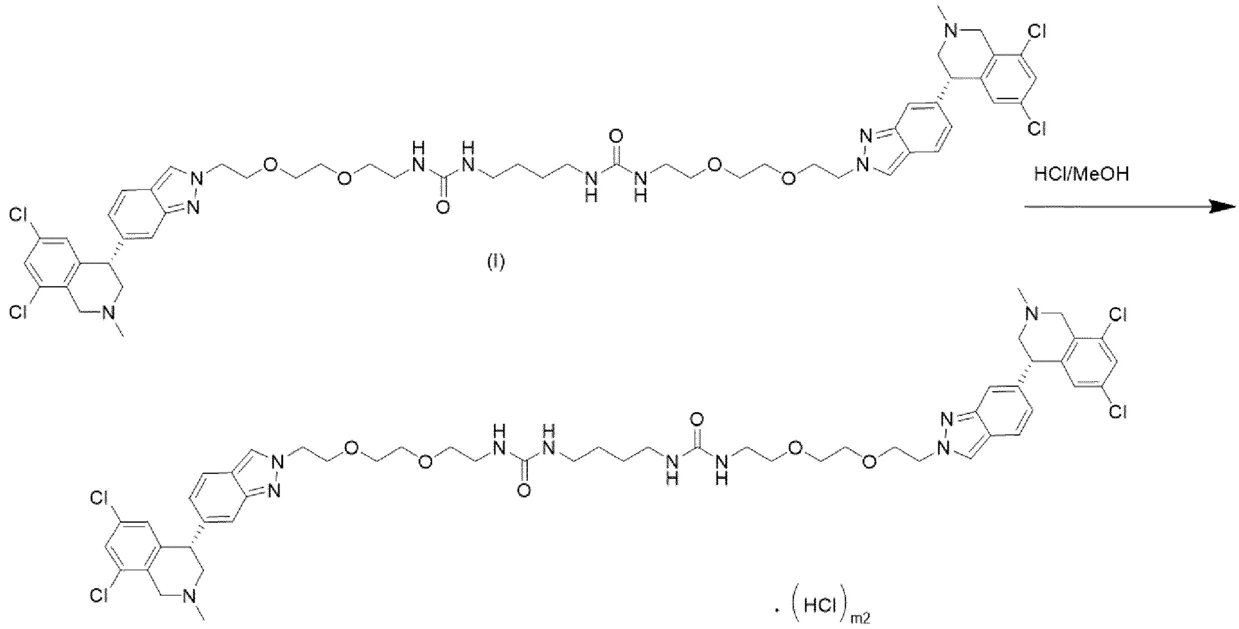


(I)

(III-2)

,

wherein m2 = 3.8-4.2, and a reaction solvent is methanol.

In some embodiments of the present disclosure, the method described above may further comprise at least one of the following additional technical features.

In some embodiments of the present disclosure, the reaction described above is performed under nitrogen atmosphere. In some embodiments of the present disclosure, the feeding molar ratio of the compound represented by formula (I) described above to ethyl acetate-hydrogen chloride is (2-10):(1-5).

In some embodiments of the present disclosure, the feeding molar ratio of the compound represented by formula (I) described above to ethyl acetate-hydrogen chloride is 2:1, or 3:1, or 4:1, or 5:1, or 6:1, or 7:1, or 8:1, or 9:1, or 10:1, or 3:2, or 4:3, or 5:3, or 5:2, or 5:4, or 6:5, or 7:2, or 7:3, or 7:4, or 7:5, or 7:6, or 8:3, or 8:5, or 8:7, or 9:2, or 9:4, or 9:5, or 9:7, or 9:8, or 10:3, or 10:7, or 10:9.

In some embodiments of the present disclosure, the molar volume ratio of the compound represented by formula (I) described above to methyl *tert*-butyl ether is (5-15) mmoL:(50-150) mL.

In some embodiments of the present disclosure, the molar volume ratio of the compound represented by formula (I) described above to methyl *tert*-butyl ether is 5 mmoL:50 mL, or 6 mmoL:100 mL, or 7 mmoL:100 mL, or 8 mmoL:100 mL, or 9 mmoL:100 mL, or 9.3 mmoL:100 mL, or 10 mmoL:100 mL, or 11 mmoL:100 mL, or 12 mmoL:100 mL, or 13 mmoL:100 mL, or 14 mmoL:150 mL, or 15 mmoL:150 mL.

In some embodiments of the present disclosure, the method further comprises a stirring treatment, a suction filtration treatment, and a drying treatment after the reaction described above is completed.

In some embodiments of the present disclosure, the stirring treatment described above is performed at room temperature.

In some embodiments of the present disclosure, the stirring treatment described above is performed at room temperature for two days.

In some embodiments of the present disclosure, the suction filtration treatment described above is performed under nitrogen atmosphere.

In some embodiments of the present disclosure, the drying treatment described above is performed under a vacuum condition at room temperature for 2 h.

In some embodiments of the present disclosure, the molar ratio of the compound represented by formula (I) described above to hydrochloric acid is 1.0:(1.6-2.1).

In another aspect of the present disclosure, the present disclosure further provides an amorphous form of a hydrochloride of a compound represented by formula (I), wherein the molar ratio of the compound represented by formula (I) to hydrochloric acid is 1.0:(1.6-4.2).

In some embodiments of the present disclosure, the amorphous form of the hydrochloride described above is selected from an amorphous form of a monohydrochloride, an amorphous form of a dihydrochloride, an amorphous form of a trihydrochloride, and an amorphous form of a tetrahydrochloride. The inventors have found that the amorphous form of the tetrahydrochloride has a higher stability and is less prone to be oxidated than other amorphous forms.

In some embodiments of the present disclosure, the molar ratio of the compound represented by formula (I) to hydrochloric acid is 1.0:1.6, 1.0:1.8, 1.0:2.1, 1.0:2.5, 1.0:2.8, 1.0:3.1, 1.0:3.5, 1.0:3.8, and 1.0:4.2.

In some embodiments of the present disclosure, when the amorphous form described above is the dihydrochloride (i.e., the molar ratio of the compound represented by formula (I) to hydrochloric acid is 1.0:(1.6-2.1), the amorphous form of the hydrochloride described above has an XPRD pattern substantially as shown in FIG. 3 or FIG. 11.

In some embodiments of the present disclosure, when the amorphous form described above is the tetrahydrochloride (i.e., the molar ratio of the compound represented by formula (I) to hydrochloric acid is 1.0:(2.2-4.2), the amorphous form of the hydrochloride described above has an XPRD pattern substantially as shown in FIG. 15.

In some embodiments of the present disclosure, when the amorphous form described above is the dihydrochloride (i.e., the molar ratio of the compound represented by formula (I) to hydrochloric acid is 1.0:(1.6-2.1), the amorphous form of the hydrochloride described above has a TGA/mDSC substantially as shown in FIG. 12.

In some embodiments of the present disclosure, when the amorphous form described above is the dihydrochloride (i.e., the molar ratio of the compound represented by formula (I) to hydrochloric acid is 1.0:(1.6-2.1), the amorphous form of the hydrochloride described above has a $^1H$ NMR spectrum substantially as shown in FIG. 13 or FIG. 14.

In some embodiments of the present disclosure, when the amorphous form described above is the tetrahydrochloride (i.e., the molar ratio of the compound represented by formula (I) to hydrochloric acid is 1.0:(2.2-4.2), the amorphous form of the hydrochloride described above has a $^1H$ NMR spectrum substantially as shown in FIG. 16.

In another aspect of the present disclosure, the present disclosure further provides a method for preparing an amorphous form of a dihydrochloride of a compound represented by formula (I), wherein the method comprises performing a stirring treatment, a suction filtration treatment, and a drying treatment on the compound represented by formula (I) and ethyl acetate-hydrogen chloride in methyl *tert*-butyl ether to obtain the amorphous form of the dihydrochloride of the compound represented by formula (I).

In another aspect of the present disclosure, the present disclosure further provides a method for preparing an amorphous form of a dihydrochloride of a compound represented by formula (I), wherein the method comprises performing a stirring treatment on the compound represented by formula (I) and ethyl acetate-hydrogen chloride in methyl *tert*-butyl ether, then performing a suction filtration treatment under nitrogen atmosphere, and then performing a vacuum drying treatment at room temperature to obtain the amorphous form of the dihydrochloride of the compound represented by formula (I).

In some embodiments of the present disclosure, the feeding molar ratio of the compound represented by formula (I) described above to ethyl acetate-hydrogen chloride is (2-10):(1-5).

In some embodiments of the present disclosure, the feeding molar ratio of the compound represented by formula (I) described above to ethyl acetate-hydrogen chloride is 2:1, or 3:1, or 4:1, or 5:1, or 6:1, or 7:1, or 8:1, or 9:1, or 10:1, or 3:2, or 4:3, or 5:3, or 5:2, or 5:4, or 6:5, or 7:2, or 7:3, or 7:4, or 7:5, or 7:6, or 8:3, or 8:5, or 8:7, or 9:2, or 9:4, or 9:5, or 9:7, or 9:8, or 10:3, or 10:7, or 10:9.

In some embodiments of the present disclosure, the molar volume ratio of the compound represented by formula (I) described above to methyl *tert*-butyl ether is (5-15) mmoL:(50-150) mL.

In some embodiments of the present disclosure, the molar volume ratio of the compound represented by formula (I) described above to methyl *tert*-butyl ether is 5 mmoL:50 mL, or 6 mmoL:100 mL, or 7 mmoL:100 mL, or 8 mmoL:100 mL, or 9 mmoL:100 mL, or 9.3 mmoL:100 mL, or 10 mmoL:100 mL, or 11 mmoL:100 mL, or 12 mmoL:100 mL, or 13 mmoL:100 mL, or 14 mmoL:150 mL, or 15 mmoL:150 mL.

In some embodiments of the present disclosure, the stirring treatment described above is performed at room temperature.

In some embodiments of the present disclosure, the stirring treatment described above is performed at room temperature for two days.

In some embodiments of the present disclosure, the suction filtration treatment described above is performed under nitrogen atmosphere.

In some embodiments of the present disclosure, the drying treatment described above is performed under a vacuum condition at room temperature for 2 h.

In some embodiments of the present disclosure, the molar ratio of the compound represented by formula (I) described above to hydrochloric acid is 1.0:(1.6-2.1).

In another aspect of the present disclosure, the present disclosure further provides a method for preparing an amorphous form of a tetrahydrochloride of a compound represented by formula (I), wherein the method comprises dropwise adding an HCl/MeOH solution to the compound represented by formula (I) in a methanol solvent under nitrogen atmosphere, and performing a stirring treatment, a concentration treatment, a slurrying treatment, a filtration treatment, and a drying treatment to obtain the amorphous form of the tetrahydrochloride of the compound represented by formula (I).

In another aspect of the present disclosure, the present disclosure further provides a method for preparing an amorphous form of a tetrahydrochloride of a compound represented by formula (I), wherein the method comprises: dissolving the compound represented by formula (I) in anhydrous methanol, dropwise adding an HCl/MeOH solution

under nitrogen atmosphere, performing a stirring treatment after the addition, then concentrating the reaction solution to obtain a crude product, and performing a slurring treatment and a filtration treatment on the crude product to obtain the amorphous form of the tetrahydrochloride of the compound represented by formula (I).

In some embodiments of the present disclosure, methanol described above is anhydrous methanol.

In some embodiments of the present disclosure, the feeding molar ratio of the compound represented by formula (I) described above to HCl/MeOH is (1-5):(2-10).

In some embodiments of the present disclosure, the feeding molar ratio of the compound represented by formula (I) described above to HCl/MeOH is 1:2, or 1:2.5, or 1:3, or 1:4, or 1:4.25, or 1:4.5, or 1:5, or 1:6, or 1:7, or 1:8, or 1:9, or 1:10, or 2:3, or 2:5, or 2:7, or 2:9, or 3:4, or 3:5, or 3:7, or 3:8, or 3:9, or 4:5, or 4:7, or 4:9, or 5:6, or 5:7, or 5:8, or 5:9, etc.

In some embodiments of the present disclosure, the mass volume ratio of the compound represented by formula (I) described above to methanol is (1-5) kg:(10-50) L.

In some embodiments of the present disclosure, the mass volume ratio of the compound represented by formula (I) described above to methanol is 4.05 kg:40 L.

In some embodiments of the present disclosure, the stirring treatment described above is performed for 30 min.

In some embodiments of the present disclosure, the slurrying treatment described above is performed in ethyl acetate for 2 h.

In some embodiments of the present disclosure, the molar ratio of the compound represented by formula (I) described above to hydrochloric acid is 1.0:(2.9-4.2).

In another aspect of the present disclosure, the present disclosure further provides use of the amorphous form of the compound represented by formula (I) described above, or the 1,5-naphthalenedisulfonate of the compound represented by formula (I) described above, or the 1,5-naphthalenedisulfonate of the compound represented by formula (I) prepared by the method described above, or the hydrochloride of the compound represented by formula (I) described above, or the compound represented by formula (III-1) prepared by the method described above, or the compound represented by formula (III-2) prepared by the method described above, or the amorphous form of the 1,5-naphthalenedisulfonate of the compound represented by formula (I) described above, or the amorphous form of the 1,5-naphthalenedisulfonate of the compound represented by formula (I) prepared by the method described above, or the amorphous form of the hydrochloride of the compound represented by formula (I) described above, or the amorphous form of the dihydrochloride of the compound represented by formula (I) prepared by the method described above, or the amorphous form of the tetrahydrochloride of the compound represented by formula (I) prepared by the method described above for a medicament for the inhibition of NHE-mediated antiport of a sodium ion or a hydrogen ion.

In another aspect of the present disclosure, the present disclosure further provides use of the amorphous form of the compound represented by formula (I) described above, or the 1,5-naphthalenedisulfonate of the compound represented by formula (I) described above, or the 1,5-naphthalenedisulfonate of the compound represented by formula (I) prepared by the method described above, or the hydrochloride of the compound represented by formula (I) described above, or the compound represented by formula (III-1) prepared by the method described above, or the compound represented by formula (III-2) prepared by the method described above, or the amorphous form of the 1,5-naphthalenedisulfonate of the compound represented by formula (I) described above, or the amorphous form of the 1,5-naphthalenedisulfonate of the compound represented by formula (I) prepared by the method described above, or the amorphous form of the hydrochloride of the compound represented by formula (I) described above, or the amorphous form of the dihydrochloride of the compound represented by formula (I) prepared by the method described above, or the amorphous form of the tetrahydrochloride of the compound represented by formula (I) prepared by the method described above for manufacturing a medicament for the treatment of a disease selected from irritable bowel syndrome, heart failure, chronic kidney disease, end-stage renal disease, and liver disease.

## Definitions and Description

Unless otherwise stated, all technical and scientific terms used herein have the same meaning as those commonly understood by those of ordinary skill in the art to which the present disclosure belongs. All patents and publications referred to herein are incorporated herein by reference in their entirety. Although any methods and materials similar or equivalent to those described herein can be used in the practice or testing of the present disclosure, preferred methods, devices, and materials are described herein.

"Crystal form" or "crystalline form" refers to a solid having a highly regular chemical structure, including, but not limited to, mono- or multi-component crystals, and/or polymorphs, solvates, hydrates, clathrates, cocrystals, and salts of compounds, and solvates of the salts or hydrates of the salts. Crystalline forms of a substance can be obtained by a number of methods known in the art. Such methods include, but are not limited to, melt crystallization, melt cooling, solvent crystallization, crystallization in a defined space, e.g., in a nanopore or capillary, crystallization on a surface or template, e.g., on a polymer, crystallization in the presence of an additive such as a co crystal counter-molecule, desolvation, dehydration, rapid evaporation, rapid cooling, slow cooling, vapor diffusion, sublimation, reactive crystallization, anti-solvent addition, grinding, solvent drop grinding, and the like.

"Amorphous" or "amorphous form" refers to a substance formed when particles (molecules, atoms, or ions) of the substance are in an aperiodic arrangement in a three-dimensional space, which is characterized by a diffuse X-ray powder diffraction pattern without sharp peaks. Amorphous is a special physical form of a solid substance, and its locally ordered structural features suggest that it is inextricably linked with a crystalline substance. Amorphous forms of a substance can be obtained by a number of methods known in the art. Such methods include, but are not limited to, quenching, anti-solvent flocculation, ball-milling, spray drying, lyophilization, wet granulation, solid dispersion techniques, and the like.

"Solvent" refers to a substance (typically a liquid) that is capable of completely or partially dissolving another substance (typically a solid). Solvents used in the present disclosure include, but are not limited to, water, acetic acid, acetone, acetonitrile, benzene, chloroform, tetrachloromethane, dichloromethane, dimethylsulfoxide, 1,4-dioxane, ethanol, ethyl acetate, butanol, *tert*-butanol, *N,N*-dimethylacetamide, *N,N*-dimethylformamide, formamide, formic acid, heptane, hexane, isopropanol, methanol, methyl ethyl ketone, l-methyl-2-pyrrolidone, mesitylene, nitromethane, polyethylene glycol, propanol, 2-acetone, pyridine, tetrahydrofuran, toluene, xylene, a mixture thereof, and the like.

"Anti-solvent" refers to a fluid that facilitates precipitation of a product (or product precursor) from a solvent. The anti-solvents may include a cold gas, or a fluid that facilitates precipitation by a chemical reaction, or a fluid that reduces the solubility of the product in the solvent. The anti-solvents may be the same liquid as the solvent having a different temperature, or may be a different liquid from the solvent.

"Solvate" refers to a crystal having a solvent on the surface, or in the crystal lattice, or on the surface and in the crystal lattice, wherein the solvent may be water, acetic acid, acetone, acetonitrile, benzene, chloroform, tetrachloromethane, dichloromethane, dimethylsulfoxide, 1,4-dioxane, ethanol, ethyl acetate, butanol, tert-butanol, *N,N*-dimethylacetamide, *N*,*N*-dimethylformamide, formamide, formic acid, heptane, hexane, isopropanol, methanol, methyl ethyl ketone, methylpyrrolidone, mesitylene, nitromethane, polyethylene glycol, propanol, 2-acetone, pyridine, tetrahydrofuran, toluene, xylene, a mixture thereof, and the like. A specific example of the solvate is a hydrate, wherein the solvent on the surface, or in the crystal lattice, or on the surface and in the crystal lattice is water. The hydrate may or may not have other solvents than water on the surface, or in the crystal lattice, or on the surface and in the crystal lattice of the substance.

Crystalline or amorphous forms may be identified by a variety of techniques, such as X-ray powder diffraction (XRPD), infrared absorption spectroscopy (IR), melting point method, differential scanning calorimetry (DSC), thermogravimetric analysis (TGA), nuclear magnetic resonance method, Raman spectroscopy, X-ray monocrystal diffraction, dissolution calorimetry, scanning electron microscopy (SEM), quantitative analysis, solubility, dissolution rate, and the like.

X-ray powder diffraction (XRPD) can detect information such as changes in crystalline forms, crystallinity, crystal state, and the like, and is a common means for identifying crystalline forms. The peak positions of XRPD patterns mainly depend on the structure of the crystalline form, being relatively insensitive to experimental details, while their relative peak heights depend on a number of factors related to sample preparation and instrument geometric shape. Accordingly, in some examples, the crystalline form of the present disclosure is characterized by an XRPD pattern having certain peak positions, as substantially shown in the XRPD patterns provided in the drawings of the present disclosure. Meanwhile, the measure of 2θ in the XRPD patterns may have experimental errors and vary slightly from instrument to instrument and from sample to sample, and therefore, the 2θ values cannot be considered absolute. The diffraction peaks have an error margin of ±0.2° according to the condition of the instrument used in the test of the present disclosure.

Differential scanning calorimetry (DSC) is a technique for measuring the energy difference between a sample and an inert reference (commonly $\alpha$-$Al_2O_3$) as a function of temperature by continuously heating or cooling under programmed control. The melting peak height of DSC curves depends on a number of factors related to sample preparation and instrument geometric shape, while peak positions are relatively insensitive to experimental details. Accordingly, in some examples, the crystalline form described herein is characterized by a DSC profile having characteristic peak positions, as substantially shown in the DSC profiles provided in the drawings of the present disclosure. Meanwhile, DSC profiles may have experimental errors, and the peak positions and peak values in the DSC profiles may vary slightly from instrument to instrument and from sample to sample. Thus, the peak positions or peak values of endothermic peaks in the DSC cannot be considered absolute. The melting peak has an error margin of ±3 °C according to the condition of the instrument used in the test of the present disclosure.

Glass transition refers to a transition of an amorphous substance between the rubbery state and the glassy state, which is an inherent property of the substance; the corresponding transition temperature is glass transition temperature (Tg), which is an important physical property of the amorphous substance. Glass transition is a phenomenon related to molecular motion. Therefore, the glass transition temperature (Tg) mainly depends on the structure of a substance, and is relatively insensitive to experimental details. In some examples, the glass transition temperature (Tg) of the amorphous form described herein is determined by differential scanning calorimetry (DSC), and the amorphous form is characterized by having a glass transition temperature of 107.44 °C. The glass transition temperature has an error margin of ±3 °C according to the condition of the instrument used in the test of the present disclosure.

Differential scanning calorimetry (DSC) can also be used for detecting and analyzing whether there is a

crystalline form transition or mixed crystal phenomenon in the crystalline form.

Solids having identical chemical composition usually form polymorphs, or variants, having different crystal structures under different thermodynamic conditions, and this phenomenon is called polymorphism. When conditions of temperature and pressure are changed, the variants are converted into each other, which is called crystalline form transition phenomenon. Due to the crystalline form transition, the mechanical, electrical, magnetic, and other properties of the crystal can be changed greatly. When the temperature of the crystalline form transition is in a measurable range, the transition process can be observed in a differential scanning calorimetry (DSC) profile, and the DSC profile has an exothermic peak reflecting the transition process and two or more endothermic peaks that are characteristic endothermic peaks of different crystalline forms before and after the transition, respectively. The crystalline form or amorphous form of the compound of the present disclosure may undergo a crystalline form transition under suitable conditions.

Thermogravimetric analysis (TGA) is a technique for measuring the change in mass of a substance as a function of temperature under programmed control, which is suitable for detecting the process of the solvent loss in a crystal or the sublimation and dissociation of a sample, thereby speculating the condition of water of crystallization or a crystallization solvent contained in the crystal. The change in mass shown in the TGA curve depends on a number of factors such as sample preparation and instrument, and varies slightly from instrument to instrument and from sample to sample. In some examples, crystalline form A of the calcium salt described herein has a weight loss of about 5.1% at a temperature of about 150 °C. The change in mass has an error margin of ±0.3% according to the condition of the instrument used in the test of the present disclosure.

In the context of the present disclosure, 2θ values in X-ray powder diffraction patterns are all expressed in degrees (°).

It should be noted that "wt%" refers to a mass ratio (g/g). For example, in a hydrate, the water content of crystalline form A is 3.0 wt%, which means that the ratio of the mass of water in the crystalline form A to the mass of the crystalline form A (g/g) is 3.0; for another example, in a solvate, the content of 1,4-dioxane in crystalline form C is 3.1 wt%, which means that the ratio of the mass of 1,4-dioxane in the crystalline form C to the mass of the crystalline form C (g/g) is 3.1.

The term "substantially as shown in the figure" means that at least 50%, or at least 60%, or at least 70%, or at least 80%, or at least 90%, or at least 95%, or at least 99% of the peaks in the X-ray powder diffraction pattern or DSC profile or TGA result are shown in the figure.

When referring to a spectrum and/or data appearing in a pattern, "peak" refers to a feature that can be recognized by those skilled in the art and will not be assigned to background noise.

"Substantially pure" means that a crystalline form is substantially free of one or more other crystalline forms, i.e., the crystalline form has a purity of at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%; or the crystalline form contains other crystalline forms, and the percentage of the other crystalline forms in the total volume or total weight of the crystalline form is less than 20%, or less than 10%, or less than 5%, or less than 3%, or less than 1%, or less than 0.5%, or less than 0.1%, or less than 0.01%.

"Substantially free of" means that the percentage of one or more other crystalline forms in the total volume or total weight of the crystalline form is less than 20%, or less than 10%, or less than 5%, or less than 4%, or less than 3%, or less than 2%, or less than 1%, or less than 0.5%, or less than 0.1%, or less than 0.01%.

"Relative intensity" refers to a ratio of the intensity of the other peaks to the intensity of the most intense peak among all diffraction peaks of an X-ray powder diffraction (XRPD) pattern where the intensity of the most intense peak is considered as 100%.

In the context of the present disclosure, when the word "about" or "approximately" is used or regardless of whether the word is used, the word means within 10%, suitably within 5%, and particularly within 1% of a given value or range. Alternatively, the term "about" or "approximately" means within an acceptable standard error of the mean for those of ordinary skill in the art. Whenever a number N is disclosed, any number within the values of N+/-1%, N+/-2%, N+/-3%, N+/-5%, N+/-7%, N+/-8%, or N+/-10% is explicitly disclosed, wherein "+/-" refers to addition or subtraction.

The term "comprise", "comprises", or "comprising" is open-ended, i.e., including what is meant by the present disclosure, but not excluding other aspects.

"Free base" refers to a form in which the compound represented by formula (I) is not a salt.

X-ray powder diffraction (XRPD) is acquired on an X-ray powder diffraction analyzer manufactured by PANalytacal with scanning parameters shown in Table 1.

Table 1

| Model | Empyrean |
|---|---|
| X-ray | Cu, Kα, Kα1 (Å):1.540598, Kα2 (Å):1.544426<br>Kα2/Kα1 intensity ratio:0.50 |

(continued)

| Model | Empyrean |
|---|---|
| X-ray light tube settings | 45 kV, 40 mA |
| Divergence slit | 1/8° |
| Scanning mode | Continuous |
| Scanning range (°2Theta) | 3-40 |
| Scanning time per step (s) | 17.8 |
| Scanning step length (°2Theta) | 0.0263 |
| Test time | about 5 min |

TGA and mDSC profiles are acquired on a TA 5500 thermogravimetric analyzer and a TA 2500 differential scanning calorimeter, respectively, and the test parameters are listed in Table 2.

Table 2

| Parameter | TGA | mDSC |
|---|---|---|
| Method | Linear heating | Linear heating |
| Sample tray | Aluminum tray, open | Aluminum tray, closed |
| Temperature range | Room temperature-setting endpoint temperature | -20 °C-setting endpoint temperature |
| Purging rate (°C/min) | 10 | 3 |
| Cycle | / | 60 s |
| Modulation amplitude (°C) | / | ± 1.0 |
| Protective gas | Nitrogen | Nitrogen |

Dynamic vapor sorption (DVS) curves are acquired on a DVS IntrInsic from Surface Measurement Systems (SMS). The relative humidity at 25 °C is corrected with the deliquescence points of LiCl, $Mg(NO_3)_2$, and KCl. The DVS test parameters are listed in Table 3.

Table 3

| Parameter | Set value |
|---|---|
| Temperature | 25 °C |
| Sample amount | 10-20 mg |
| Protective gas and flow rate | $N_2$, 200 mL/min |
| dm/dt | 0.002%/min |
| Minimum dm/dt equilibration time | 10 min |
| Maximum equilibration time | 180 min |
| RH range | 0%RH-95%RH |
| RH gradient | 10%RH (0%RH to 90%RH & 90%RH to 0%RH)<br>5%RH (90%RH to 95%RH & 95%RH to 90%RH) |

The liquid-state nuclear magnetic resonance spectra are acquired on a Bruker 400M nuclear magnetic resonance spectrometer with DMSO-$d_6$ as a solvent.

In high performance liquid chromatography and ion chromatography (HPLC/IC):
in the test, the molar ratio and stability are tested using an Agilent 1260 high performance liquid chromatograph, the molar ratio of salt-forming ions is tested using ion chromatography, and the analytical conditions are shown in Table 4 and Table 5.

Table 4

| | | | | |
|---|---|---|---|---|
| Liquid chromatograph | Agilent 1260/1290 detector | | | |
| Chromatographic column | Waters Xbridge, C18, 4.6 mm/150 mm/5 µm | | | |
| Mobile phase | A:10 mM $NH_4HCO_3$ in $H_2O$ | | | |
| | B: Acetonitrile | | | |
| Elution gradient | Molar ratio | | Purity | |
| | Time (min) | %B | Time (min) | %B |
| | 0.0 | 40 | 0.0 | 40 |
| | 10.0 | 95 | 24.0 | 95 |
| | 12.0 | 95 | 27.0 | 95 |
| | 12.1 | 40 | 27.1 | 40 |
| | 15.0 | 40 | 30.0 | 40 |
| Run time | 15 min | | 30 min | |
| Flow rate of mobile phase | 1.0 mL/min | | | |
| Injection volume | 5 µL | | | |
| Detection wavelength | UV at 220 nm | | | |
| Column temperature | 40 °C | | | |
| Injector temperature | RT | | | |
| Diluent | Acetonitrile | | | |

Table 5

| | |
|---|---|
| Ion chromatograph | ThermoFisher ICS-1100 |
| Chromatographic column | IonPac AS18 Analytical Column, 250*4 mm |
| Mobile phase | 25 mM NaOH |
| Injection volume | 25 µL |
| Flow rate | 1.0 mL/min |
| Temperature | 35 °C |
| Column temperature | 35 °C |
| Current | 80 mA |
| Run time | $Cl^-$: 7 min |

The test conditions for HPLC during the preparation of the tetrahydrochloride are shown in Table 6 below.

Table 6

| Name | Parameter |
|---|---|
| Instrument | High performance liquid chromatograph |
| Chromatographic column | Waters XBridge C18 (150 mm* 4.6 mm, 3.5 µm) |
| Mobile phase A | 10 mM aqueous ammonium acetate solution (pH = 5±0.1) |
| Mobile phase B | Acetonitrile |
| Flow rate | 1.0 mL/min |
| Detector | UV 282 nm |
| Injection volume | 20 µL |

(continued)

| | | | |
|---|---|---|---|
| Column temperature | 40 °C | | |
| Run time | 23.00 min | | |
| Elution program | Time (min) | A (%) | B (%) |
| | 0.00 | 60 | 40 |
| | 8.00 | 42 | 58 |
| | 15.00 | 5 | 95 |
| | 18.00 | 5 | 95 |
| | 18.10 | 60 | 40 |
| | 23.00 | 60 | 40 |

The instrument for determining hydrochloric acid by titration is shown in Table 7 below.

Table 7

| Name | Parameter |
|---|---|
| Instrument | Potentiometric titrator |
| Electrode | Metallic silver electrode, Switzerland Metrohm PN: 6.00430.100 |
| Titration mode | DET U |
| Stirring speed | 5 |
| Starting condition | Pause for 60 s |
| Starting condition | 15 mL |

Test method: solution preparation:

(1) Blank solution: 80 mL of purified water is measured and placed in a 200 mL beaker, 10 mL of a nitric acid solution is added, and then 20 mL of an acetonitrile solution is added.
(2) Test sample solution: about 100 mg of a sample is precisely weighed and placed in a 200 mL beaker, about 80 mL of a diluent is added, 10 mL of a nitric acid solution is added, and then 20 mL of an acetonitrile solution is added (acetonitrile is added for assisting dissolution because the sample is not easily dissolved in a mixed solution of purified water and nitric acid). The mixed solution is shaken well and sonicated for dissolution. The steps are repeated from weighing, and the solution is prepared in duplicate.

## BRIEF DESCRIPTION OF THE DRAWINGS

FIG. 1 is an XPRD pattern of an amorphous form of a free base of a compound represented by formula (I);

FIG. 2 is an XPRD pattern of a 1,5-naphthalenedisulfonate of the compound represented by formula (I);

FIG. 3 is an XPRD pattern of dihydrochloride sample 1 of the compound represented by formula (I);

FIG. 4 is an XPRD pattern of 1,5-naphthalenedisulfonate sample 1 of the compound represented by formula (I);

FIG. 5 is a TGA/mDSC of 1,5-naphthalenedisulfonate sample 1 of the compound represented by formula (I);

FIG. 6 is a $^1$H NMR spectrum of 1,5-naphthalenedisulfonate sample 1 of the compound represented by formula (I);

FIG. 7 is an XPRD pattern of 1,5-naphthalenedisulfonate sample 2 of the compound represented by formula (I);

FIG. 8 is a TGA/mDSC of 1,5-naphthalenedisulfonate sample 2 of the compound represented by formula (I);

FIG. 9 is a $^1$H NMR spectrum of 1,5-naphthalenedisulfonate sample 2 of the compound represented by formula (I);

FIG. 10 is a $^1$H NMR spectrum of a 1,5-naphthalenedisulfonate of the compound represented by formula (I) prepared repeatedly;

FIG. 11 is an XPRD pattern of dihydrochloride sample 1 of the compound represented by formula (I);

FIG. 12 is a TGA/mDSC profile of dihydrochloride sample 1 of the compound represented by formula (I);

FIG. 13 is a $^1$H NMR spectrum of dihydrochloride sample 1 of the compound represented by formula (I);

FIG. 14 is a $^1$H NMR spectrum of a dihydrochloride of the compound represented by formula (I) prepared repeatedly;

FIG. 15 is an XPRD pattern of a tetrahydrochloride sample of the compound represented by formula (I);

FIG. 16 is a $^1$H NMR spectrum of the tetrahydrochloride sample of the compound represented by formula (I);

FIG. 17 shows a normalized correction value nP for urinary phosphorus excretion amount versus dietary phosphorus intake;

FIG. 18 shows a normalized correction value nNa for urinary sodium excretion amount versus dietary sodium intake;

FIG. 19 shows a fecal form score;

FIG. 20 is an experimental process diagram;

FIG. 21 shows the serum phosphorus concentration in rats;

FIG. 22 shows a normalized correction value nP of urinary phosphorus excretion amount and dietary phosphorus intake over 24 h;

FIG. 23 shows a normalized correction value nNa of urinary sodium excretion amount and dietary sodium intake over 24 h; and

FIG. 24 shows a fecal form score.

## DETAILED DESCRIPTION

The present application is described in detail below by way of examples, but it does not mean that there are any adverse limitations on the present application. Although the present application has been described in detail herein and specific embodiments thereof have also been disclosed, it will be apparent to those skilled in the art that various changes and modifications can be made to the specific embodiments of the present application without departing from the spirit and scope of the present application.

The starting materials used in the present disclosure are commercially available unless otherwise specified.

The abbreviations or names of the solvents used in the present disclosure are shown in Table 8 below:

Table 8

| abbreviations or names | meanings | abbreviations or names | meanings |
|---|---|---|---|
| MeOH | Methanol | MTBE | Methyl *tert*-butyl ether |
| EtOH | Ethanol | THF | Tetrahydrofuran |
| IPA | Isopropanol | 2-MeTHF | 2-Methyltetrahydrofuran |
| $CHCl_3$ | Chloroform | ACN | Acetonitrile |
| MIBK | Methyl isobutyl ketone | n-Heptane | Normal heptane |

(continued)

| abbreviations or names | meanings | abbreviations or names | meanings |
|---|---|---|---|
| EtOAc | Ethyl acetate | Toluene | Methylbenzene |
| IPAc | Isopropyl acetate | $H_2O$ | Water |
| DMSO | Dimethylsulfoxide | DCM | Dichloromethane |
| Anisole | Phenyl methyl ether | 1,4-Dioxane | 1,4-Dioxane |
| MEK | Methyl ethyl ketone | DMF | Dimethylformamide |
| Cumene | Isopropyl benzene | n-Hexane | Normal hexane |

Example 1. Preparation of Compound Represented by Formula (I)

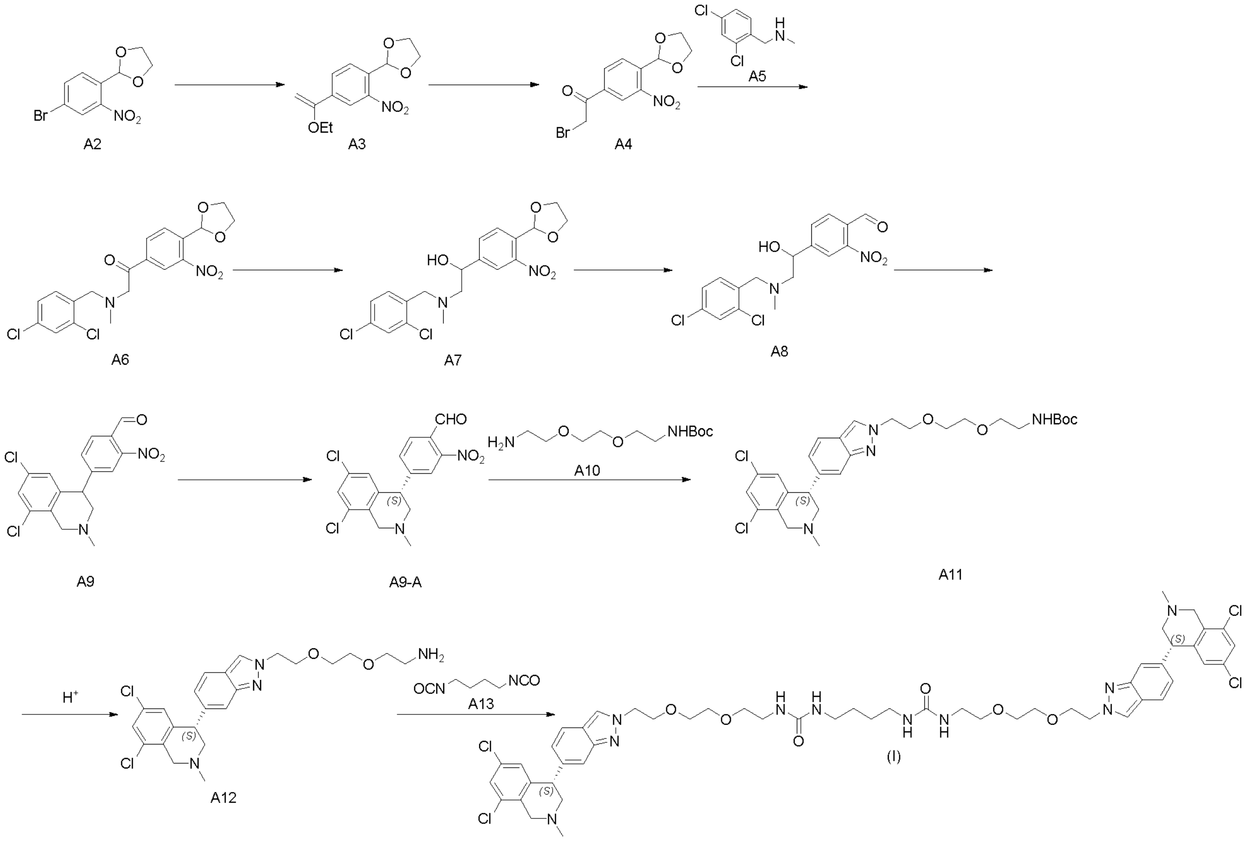


Step 1

Tributyl(1-ethoxyvinyl)tin (515 g, 1.3 mol) and Pd(dppf)$Cl_2$ (3.15 g, 857.47 mmol) were added to A2 (363 g, 1.30 mol) in dioxane (2 L) at room temperature. The reaction solution was purged with nitrogen and stirred at 100 °C for 16 h under nitrogen atmosphere. After the reaction was completed, as monitored by LCMS, the reaction was quenched with 10% KF solution (1.5 L), stirred for 1 h, diluted with ethyl acetate (3 L) and filtered. The filter cake was washed with ethyl acetate. The solution was liquid separated, and the aqueous phase was extracted with ethyl acetate (2.5 L × 2). The organic phases were combined, dried, filtered, and concentrated to give a crude product of A3 (344 g), which was used directly in the next step.

Step 2

NBS (230.8 g, 1.3 mol) was added to a solution (a mixed solvent of THF:$H_2O$ = 3:1, 2 L) of **A3** (344 g, 1.30 mol) at 0

°C. Water (1.5 L), a saturated $Na_2SO_3$ solution (200 mL), and a sodium bicarbonate solution (200 mL) were added sequentially to the reaction solution, and the resulting solution was stirred for half an hour. The aqueous phase was extracted with ethyl acetate (0.5 L × 3), and the organic phases were combined, washed with saturated brine (1 L), dried over anhydrous sodium sulfate, filtered, and concentrated to give a crude product of **A4** (380 g), which was used directly in the next step.

Step 3

**A5** (244.7 g, 1.3 mol) and DIPEA (503 g, 3.89 mol) were added to **A4** (380 g, 1.30 mol) in dioxane (2 L) at 0 °C. After the addition was completed, the reaction mixture was stirred at room temperature for one hour. After the reaction was completed, as monitored by LCMS, the reaction solution was filtered. Water (1.5 L) and ethyl acetate (2 L) were added sequentially to the filtrate for liquid separation. The aqueous phase was extracted with ethyl acetate (0.5 L × 2). The organic phases were combined, washed with saturated brine (1 L), dried over anhydrous sodium sulfate, filtered, and concentrated to give a crude product of **A6** (900 g), which was used directly in the next step.

Step 4

$NaBH_4$ (49 g, 1.3 mol) was added to **A6** (crude, 900 g, 1.30 mol) in methanol (4 L) at 0 °C. After the addition was completed, the reaction solution was stirred at 0 °C for 1 h. After the reaction was completed, as monitored by LCMS, a saturated $NH_4Cl$ solution (500 mL) was added to the reaction solution, and then a 3 N HCl solution was added to adjust the solution to neutral. The reaction solution was concentrated, and dissolved in DCM for liquid separation. The aqueous phase was extracted with DCM (0.5 L × 2). The organic phases were combined, washed with saturated brine (1 L), dried over anhydrous sodium sulfate, and concentrated. The crude product was further purified by column chromatography to give product **A7** (300 g), which was directly used in the next step.

Step 5

A 4 N HCl solution (400 mL) was added to **A7** (80 g, 187.23 mmol) in THF (400 mL). After the addition was completed, the reaction solution was stirred at 40 °C for 16 h. After the starting materials of the reaction were substantially converted, as monitored by LCMS, the reaction solution was neutralized with a saturated sodium bicarbonate solution, diluted with ethyl acetate (1 L) and liquid separated. The aqueous phase was extracted with ethyl acetate (0.8 L × 2). The organic phases were combined, washed with saturated brine (800 mL), dried over anhydrous sodium sulfate, and concentrated to give product **A8** (75 g), which was directly used in the next step.

Step 6

$CF_3SO_3H$ (120 mL) was added to **A8** (60 g, 156.6 mmol) in DCE (600 mL). After the addition was completed, the reaction mixture was stirred at 65 °C for 24 h. After most of the starting materials of the reaction were converted, as monitored by LCMS, the reaction solution was slowly poured into an ice water bath, and neutralized with a saturated sodium bicarbonate solution. The aqueous phase was extracted with DCM (0.5 L × 3), and the combined organic phase was dried over anhydrous sodium sulfate, concentrated, and separated by silica gel column chromatography to give product **A9** (33 g).

Step 7

**A9** (100 g) was separated by chiral column chromatography to give product **A9A** (41 g) in a single configuration. A9 can also be resolved by a chemical resolution method using di-*p*-toluoyl-*L*-tartaric acid as a resolving agent.

Salification in chemical resolution method: about 8.4 g of racemate **A9** (A9-CHP4075) was weighed out and dissolved in acetone (210 mL). About 800 mg of crystal seed (**A9A**: (D) (+)-p-methyldibenzoyltartaric acid = 1:1) was added. 1.1 equivalents of (D) (+)-*p*-methyldibenzoyltartaric acid was weighed out and dissolved in acetone (70 mL). The acetone solution in which the ligand was dissolved was slowly added dropwise to the racemate solution (the time for the dropwise addition was 4 h), suspended and stirred at room temperature for 3 days. The solid was separated by suction filtration under vacuum, and the obtained solid was slurried and washed with acetone (35 mL) for about 30 min. The ee value of the obtained solid was 97.2%, the impurity configuration concentration in the supernatant was 3.2 mg/mL, and the target configuration concentration was 3.7 mg/mL.

The solid was separated by suction filtration under vacuum again, and the obtained solid was slurried and washed with acetone (50 mL) for 30 min. The ee value of the obtained solid was 97.9%, the impurity configuration concentration in the supernatant was 0.3 mg/mL, and the target configuration concentration was 3.5 mg/mL. Finally, the

solid (6.9 g, 823325-43-C) obtained by salification had an ee value of 97.9% and a yield of about 35%.

Purification/dissociation: disodium hydrogen phosphate (50 g) was taken and added to water (1000 mL), and the resulting mixture was stirred to be clear for later use. The solid obtained by salification (100 g, 0.13 mol) was added to dichloromethane (1000 mL), and the prepared disodium hydrogen phosphate solution was slowly added dropwise at room temperature. After the dropwise addition was completed, the mixture was stirred for 2-3 h, and liquid separated. The aqueous phase was extracted twice with dichloromethane (250 mL). The organic phases were combined and washed twice with water (500 mL). The dichloromethane phase was concentrated under reduced pressure to remove water, and supplemented to 1500 mL after the removal of water was completed. 200-300 mesh silica gel (50 g) was added, and the mixture was stirred for 0.5-1 h and filtered through a sand core funnel. The silica gel was rinsed with dichloromethane (500-1000 mL), and the filtrate was concentrated under reduced pressure to give an oil. Methyl *tert*-butyl ether (200 mL-500 mL) was added, and the mixture was further concentrated to dryness under reduced pressure to give a yellow solid. Ethyl acetate/n-heptane (500 mL, v/v = 1:10) was added, and the mixture was slurried overnight and filtered to give A9A as a light yellow solid.

Step 8

**A9A** (100.0 g, 0.27 mol), EtOH (500 mL), and **A10** (74.8 g, 0.30 mol) were added to a 2 L three-necked flask at room temperature, stirred, heated to 70 °C and reacted for 4 h under $N_2$ atmosphere. In-process control of reaction: HPLC showed starting material **A9A** ≤ 5.00% (254 nm). After the in-process control was qualified, the mixture was cooled to 20-30 °C, and tri-n-butylphosphine (163.9 g, 0.81 mol) was added to the reaction flask. The resulting mixture was heated to 70 °C and further stirred for about 12-18 h. For the in-process control of the reaction, imine intermediate ≤ 2.00% (254 nm). After the reaction was completed, the mixture was cooled, and the reaction solution of **A11** was directly used in the next step.

Step 9

An ethanol dilution (300 mL) of a hydrogen chloride-ethanol solution (216 mL) was slowly added dropwise, heated to 40 °C and stirred for 12-18 h, and then stirred at room temperature overnight. The reaction endpoint was monitored by HPLC: HPLC showed starting material **A11** ≤ 2% (254 nm). The reaction solution was concentrated to dryness under reduced pressure, and dichloromethane (1 L) was added in two portions. The resulting mixture was concentrated to dryness under reduced pressure, and water (1 L) was added for dissolution. The resulting solution was extracted with ethyl acetate (500 mL), and the extraction was repeated 4 times. The aqueous phase was cooled to 0-5 °C. A 20% aqueous sodium hydroxide solution was slowly added dropwise until a pH greater than 12, and dichloromethane (1 L) was added for extraction. The aqueous phase was extracted with dichloromethane (500 mL), and the organic phases were combined and concentrated to dryness.

Purification: the crude product was dissolved with ethyl acetate (500 mL), and subjected to a wet column chromatography (100-200 mesh silica gel, 1 kg silica gel). After the impurity point above the product point was washed off with ethyl acetate:methanol = 4:1, the column was rinsed with dichloromethane:methanol = 5:1 (1% aqueous ammonia) until there was no product point. After concentration under reduced pressure, dichloromethane (1.5 L) was added in 3 portions, and the mixture was further concentrated to dryness under reduced pressure to give intermediate **A12** (108 g in total). The total yield of the two steps was 86.3%.

Step 10

**A12** (94.0 g, 0.20 mol) (by content) was dissolved in dichloromethane (940 mL). The reaction solution was cooled to 0-10 °C, and a solution of **A13** (14.0 g, 0.10 mol) in DCM (1.5 L) was added dropwise under nitrogen atmosphere. After the dropwise addition was completed, the mixture was heated to 20-30 °C and stirred for 2 h. The reaction endpoint was monitored by HPLC: A12 ≤ 1.0%. If it failed, the mixture was cooled to 0-10 °C and supplemented with **A13** (supplemented according to 0.5 equivalent of the residual A12 content under actual in-process control). After the supplement was completed, the mixture was heated to 20-30 °C and stirred for another 6 h, and HPLC monitoring was performed until the endpoint of the reaction. The reaction solution was concentrated under reduced pressure to give a crude product.

Purification by column chromatography: the crude product was dissolved with a small amount of ethyl acetate:methanol = 2:1, and wet loaded (100-200 mesh silica gel, 1 kg silica gel). The mixture was eluted with an eluent of ethyl acetate:methanol = 4:1 to remove the cross point, then eluted with ethyl acetate:methanol = 2:1 (about 8 L) until the product point became significantly lighter and eluted with ethyl acetate:methanol = 1:1 to flush a cross point. After the cross point was concentrated to dryness, a secondary column chromatography was performed (the same method as above). The pure products were combined and concentrated to dryness. Ethyl formate (300 mL) was added and vigorously stirred until being dispersed. Isopropyl ether (1.8 L) was quickly added dropwise, stirred for 10-30 min, filtered and washed with

isopropyl ether (200 mL). The filter cake was dried under vacuum at 40 °C to give a refined free amine product (57.0 g in total, 53.4% yield). The free product, compound represented by formula (I) had an XPRD pattern shown in FIG. 1.

**Example 2. Salt Form Screening of Compound Represented by Formula (I)**

Screening was conducted with hydrochloric acid (with a feeding molar ratio of 4:1 to the compound represented by formula (I)), sulfuric acid (with a feeding molar ratio of 2:1 to the compound represented by formula (I)), 1,5-naphthalenedisulfonic acid (with a feeding molar ratio of 1:1 to the compound represented by formula (I)), and ethyl acetate-hydrogen chloride (with a feeding molar ratio of 2:1 to the compound represented by formula (I)). The results are shown in Table 9 below.

Table 9

| Experiment No. | Solvent (v/v) | Hydrochloric acid | Sulfuric acid | 1,5-Naphthalenedisulfonic acid | Ethyl acetate-hydrogen chloride |
|---|---|---|---|---|---|
| 1 | MeOH | Clarified | Clarified | -- | -- |
| 2 | EtOH | Clarified | Gelling | -- | -- |
| 3 | MIBK | Gelling | Gelling | -- | -- |
| 4 | THF | Gelling | Gelling | -- | -- |
| 5 | IPA | -- | -- | Amorphous form | -- |
| 6 | EtOAc | -- | -- | Amorphous form | -- |
| 7 | ACN/$H_2O$(19:1) | -- | -- | Gelling | -- |
| 8 | MTBE | -- | -- | -- | Amorphous form |
| 9 | Toluene | -- | -- | -- | Gelling |
| 10 | $CHCl_3$ | -- | -- | -- | Gelling |

The inventors found that the compound represented by formula (I) can only form a 1,5-naphthalenedisulfonate and a hydrochloride.

**Example 3. 1,5-Naphthalenedisulfonate Sample**

Salt form preparation:

1,5-Naphthalenedisulfonate sample (1) was obtained as follows: the free base form of the compound represented by formula (I) (20.0 mg) and 5.6 mg of 1,5-naphthalenedisulfonic acid (with a molar ratio of 1:1, acid/base) were used as starting materials, stirred in IPA (0.5 mL) at room temperature for 2 days, subjected to suction filtration under nitrogen atmosphere, and dried under vacuum at room temperature for 2 h. The sample was a white powder. The XRPD of the sample, as shown in FIG. 4, shows an amorphous product. The TGA/mDSC results are detailed in FIG. 5. The TGA result shows a weight loss of 11.8% when the sample was heated to 150 °C; and no significant glass transition temperature was observed from the mDSC result. $^1$H NMR was measured in DMSO-$d_6$, and the results are shown in FIG. 6. The results show that the molar ratio of 1,5-naphthalenedisulfonic acid to the free base was 1.0:1, and 3.4 wt% of the residual IPA solvent was observed.

1,5-Naphthalenedisulfonate sample (2) was obtained as follows: the free base form of the compound represented by formula (I) (20.2 mg) and 5.6 mg of 1,5-naphthalenedisulfonic acid (with a molar ratio of 1:1, acid/base) were used as starting materials, stirred in EtOAc (0.5 mL) at room temperature for 2 days, subjected to suction filtration under nitrogen atmosphere, and dried under vacuum at room temperature for 2 h. The sample was a white powder. The XRPD of the sample, as shown in FIG. 7, shows an amorphous product. The TGA/mDSC results are detailed in FIG. 8. The TGA result shows a weight loss of 6.1% when the sample was heated to 150 °C; and no significant glass transition temperature was observed from the mDSC result. $^1$H NMR was measured in DMSO-$d_6$, and the results are shown in FIG. 9. The results show that the molar ratio of 1,5-naphthalenedisulfonic acid to the free base was 0.9:1, and no EtOAc solvent residue was observed.

The 1,5-naphthalenedisulfonate form was prepared repeatedly on a 200 mg scale: 199.2 mg of the free base form of the compound represented by formula (I) and 51.4 mg of 1,5-naphthalenedisulfonic acid were weighed out, and IPA (5.0 mL) was added. The mixture was suspended and stirred at room temperature for 2 days, and the suspension was

subjected to suction filtration and dried under vacuum at room temperature for 5 h. A sample (204.5 mg) was collected. The XRPD, as shown in FIG. 1, shows an amorphous product, and no oil formation or gelling was observed during the salification. $^1H$ NMR was measured in DMSO-$d_6$, and the results are shown in FIG. 10. The molar ratio of 1,5-naphthalenedisulfonic acid to the free base in the sample was 1.0:1, and 12.0 wt% of the residual IPA solvent was observed.

**Example 4. Hydrochloride Sample**

The dihydrochloride sample was obtained as follows: the free base form (20.0 mg) of the compound represented by formula (I) and an ethyl acetate-hydrogen chloride solution (with a molar ratio of 4:1, acid/base) were used as starting materials, stirred in MTBE (0.5 mL) at room temperature for 2 days, subjected to suction filtration under nitrogen atmosphere, and dried under vacuum at room temperature for 2 h. The sample was a white powder. The XRPD pattern of the sample, as shown in FIG. 11, shows an amorphous product. The TGA/mDSC results are presented in FIG. 12. The TGA result shows a weight loss of 6.82% when the sample was heated to 150 °C; and no significant glass transition temperature was observed from the mDSC result. $^1H$ NMR was measured in DMSO-$d_6$, and the results, as shown in FIG. 13, indicate that 0.2% of the residual MTBE solvent was observed in the sample. The HPLC/IC results indicate that the molar ratio for the amorphous sample of the hydrochloride was 2.1:1 (hydrochloric acid:free base). The dihydrochloride form was prepared repeatedly on a 200 mg scale: 375.2 μL of an ethyl acetate-hydrogen chloride solution (2 mol/L) was weighed out and diluted with MTBE (5.0 mL). The free base (200.1 mg) was weighed out and added to the clarified solution in step 1, and the mixture was suspended and stirred at room temperature for 2 days. The suspension was subjected to suction filtration and dried under vacuum at room temperature for 5 h. A sample (174.3 mg) was collected. The XRPD of the hydrochloride sample prepared repeatedly is shown in FIG. 2. $^1H$ NMR was measured in DMSO-$d_6$, and the results, as shown in FIG. 14, indicate that no MTBE solvent residue was observed. The HPLC/IC results indicate that the molar ratio for the hydrochloride sample was 1.6:1 (hydrochloric acid:free base).

Preparation of tetrahydrochloride sample: ethyl acetate (480 mL) and the free base (24.0 g, 22.49 mmol) were added to a 1 L three-necked flask, and cooled to 0-10 °C under nitrogen atmosphere. A hydrogen chloride-ethyl acetate solution was slowly added dropwise. After the dropwise addition was completed, the mixture was heated to 20-30 °C, vigorously stirred for 24-30 h, filtered, and washed with ethyl acetate (240 mL) (the filter cake was always kept in the ethyl acetate solution). The filter cake was returned to the flask, ethyl acetate (480 mL) was added, and the mixture was stirred for 0.5-1 h, filtered, and washed with ethyl acetate (240 mL). The filter cake was quickly taken out and put into a vacuum drying oven for vacuum drying at 50 °C for 12-24 h to give a refined product (26.2 g in total, 96.3% yield). The XRPD of the hydrochloride sample prepared is shown in FIG. 15. $^1H$ NMR was measured in DMSO-$d_6$, and the results, as shown in FIG. 16, indicate that the ethyl acetate solvent residue was observed. The results of measuring hydrochloric acid by HPLC and a titration method indicate that the molar ratio for the hydrochloride sample was 4.15:1 (hydrochloric acid:free base) (the total purity measured was 97.32%, and the content of the free base was 83% and the content of hydrochloric acid was 11.8% by quantitative nuclear magnetic assay. Calculation method: the calculation method of the hydrochloric acid molar ratio was as follows: molar quantity of hydrochloric acid: hydrochloric acid content/mw (HCl) = 11.8%/36.5 = $3.23 \times 10^{-3}$; molar quantity of the free base: free base content/mw (free base) = 83%/1066.95 = $7.78 \times 10^{-4}$; and the molar ratio of hydrochloric acid to the free base = molar quantity of hydrochloric acid/molar quantity of the free base = $3.23 \times 10^{-3}/7.8 \times 10^{-4} = 4.15$).

**Comparative Example 1. Hydrochloride Solvent Screening Test**

The inventors adopted different solvents to totally set 10 suspension stirring tests at room temperature and 10 suspension stirring tests at 50 °C. About 20 mg of each free base of the compound represented by formula (I) was weighed into an HPLC glass vial, and 0.5 mL of the solvents listed in Tables 10 and 11 were separately added. As a result, only gelling and clarified solutions were observed.

Table 10. Suspension stirring at room temperature

| Test No. | Solvent (v/v) | Result |
|---|---|---|
| 1 | EtOH/acetone (1:3) | Gelling |
| 2 | MeOH/THF (1:5) | Gelling |
| 3 | EtOH/EtOAc (1:3) | Gelling |
| 4 | EtOH/ACN (1:3) | Clarified |
| 5 | THF/$H_2O$ (3:1) | Clarified |

(continued)

| Test No. | Solvent (v/v) | Result |
|---|---|---|
| 6 | MeOH/$H_2O$ (3:1) | Clarified |
| 7 | IPA/$H_2O$ (3:1) | Clarified |
| 8 | MeOH/toluene (1:3) | Clarified |
| 9 | MeOH/IPA (1:3) | Clarified |
| 10 | Acetone/$H_2O$ (3:1) | Clarified |

Table 11. Suspension stirring at 50 °C

| Test No. | Solvent (v/v) | Result |
|---|---|---|
| 1 | MeOH/MTBE (1:5) | Gelling |
| 2 | MeOH/MEK (1:5) | Gelling |
| 3 | EtOH/MIBK (1:6) | Gelling |
| 4 | ACN/$H_2O$ (9:1) | Oil forming |
| 5 | EtOH/n-heptane (1:5) | Gelling |
| 6 | 1,4-Dioxane/$H_2O$ (9:1) | Oil forming |
| 7 | 1-PrOH/$H_2O$ (1:4) | Clarified |
| 8 | MeOH/IPAc(1:5) | Gelling |
| 9 | EtOH/acetone (1:5) | Gelling |
| 10 | EtOH/ACN (1:5) | Clarified |

**Experimental Example 1. Inhibition of Phosphate and Sodium Absorption in Intestinal Tract of Rats After Single Administration**

The compound represented by formula (I) was evaluated by measuring the concentrations of urinary phosphorus and urinary sodium, as well as fecal form.

Six-week-old Sprague-Dawley rats were purchased from Beijing Vital River Laboratory Animal Technology Co., Ltd. Two rats in each cage were placed in the SPF-grade animal room, and the rats were acclimatized for about one week. Throughout the study, the animals were given *ad libitum* access to feed and water, and the light cycle was 12 h of light and 12 h of darkness. Animal groups: vehicle group, n = 5; tenapanor 0.3 mg/kg group, n = 5; compound represented by formula (I) 0.03 mg/kg group, n = 5; compound represented by formula (I) 0.1 mg/kg group, n = 5; compound represented by formula (I) 0.3 mg/kg group, n = 5; and compound represented by formula (I) 1.0 mg/kg group, n = 5.

On the day of the experiment, the test compound or vehicle (0.5% Tween 80 + 99.5% distilled water) was administered intragastrically to the animals after 8 h of fasting. Then, the animals were transferred into metabolism cages for single-cage feeding, and the feed was restored. Sixteen hours after administration, urine samples were collected, food consumption was recorded, and fecal form in the collection funnel was evaluated by two independent observations. Fecal form scoring criteria: 1. normal pellets; 2. slightly soft feces (pellets adhered to the side wall of the collector due to moisture); 3. soft feces (loss of normal shape of the pellets); 4. loose and amorphous (complete loss of shape, accompanied by a blot pattern); and 5. diarrhea (watery feces). Fecal form score (FFS) in rats was determined by averaging two independent observation scores for all rats in the same group (n = 5); and the mean value for the vehicle group was 1.

The urine was centrifuged at 4 °C at 3220 g for 5 min, and the urinary phosphorus concentration (phosphomolybdic acid ultraviolet endpoint colorimetric method) and urinary sodium concentration (ion selective electrode method) were measured.

The results were expressed as mean ± standard error (Means±SEM). The urinary phosphorus excretion amount (or urinary sodium excretion amount) in the rats was normalized and corrected against the respective dietary phosphorus (or sodium) intake. The formula was as follows: normalized correction value of urinary phosphorus excretion amount (expressed as nP) = urinary phosphorus excretion amount ÷ dietary phosphorus intake; normalized correction value of urinary sodium excretion amount (expressed as nNa) = urinary sodium excretion amount ÷ dietary sodium intake; one-way analysis of variance was adopted; and fecal form score was verified by the non-parametric test. *, $p < 0.05$; **, $p < 0.01$; ***, $p < 0.001$; ****, $p < 0.0001$.

FIGs. 17-19 showed the effects of the compound represented by compound formula (I) on the excretion amounts of urinary phosphorus and urinary sodium, as well as fecal form in normal rats after the single administration. These results showed that the compound represented by compound formula (I) had a dose-effect relationship in reducing urinary phosphorus excretion amount, and could significantly reduce urinary phosphorus excretion amount at the doses of 0.3 mg/kg and 1.0 mg/kg. The same dose (0.3 mg/kg) of the compound represented by formula (I) was better than Tenapanor in reducing urinary phosphorus excretion amount. The compound represented by formula (I) could significantly reduce urinary sodium excretion amount. The water content in the feces of the rats had the tendency to increase with the increase in the dose of the compound represented by formula (I).

**Experimental Example 3. Effect of Multiple Administrations on Serum Phosphorus Concentration in Rats**

The compound represented by formula (I) was evaluated by measuring the serum phosphorus concentration, concentrations of urinary phosphorus and urinary sodium, and fecal form in rats.

Six-week-old Sprague-Dawley rats were purchased from Beijing Vital River Laboratory Animal Technology Co., Ltd. Two rats in each cage were placed in the SPF-grade animal room, and the rats were acclimatized for about one week. Throughout the study, the animals were given *ad libitum* access to feed and water, and the light cycle was 12 h of light and 12 h of darkness. Animal groups: vehicle group, n = 5; Tenapanor 0.05 mg/kg, n = 5; compound represented by formula (I) 0.01 mg/kg, n = 5; compound represented by formula (I) 0.05 mg/kg, n = 5; compound represented by formula (I) 0.5 mg/kg, n = 5; and compound represented by formula (I) 1.0 mg/kg, n = 5.

After the start of the experiment, the feeding rhythm of the rats was adjusted to diurnal feeding in a manner of overnight fasting for 16 h and restoring feed for 8 h during the day. After the start of administration, the test compound or vehicle (0.5% Tween 80 + 99.5% distilled water) was administered intragastrically twice a day during the feeding period, with an interval of 4 h, for 14 consecutive days. The body weight of animals and food consumption were measured daily. The serum phosphorus concentration (including baseline values before the administration), 24-hour urinary phosphorus excretion amount, and 24-hour urinary sodium excretion amount were measured 1-2 times a week, and fecal form in the collection funnel was scored (fecal form was assessed by two independent observations). Fecal form scoring criteria: 1. normal pellets; 2. slightly soft feces (pellets adhered to the side wall of the collector due to moisture); 3. soft feces (loss of normal shape of the pellets); 4. loose and amorphous (complete loss of shape, accompanied by a blot pattern); and 5. diarrhea (watery feces). Fecal form score (FFS) in rats was determined by averaging two independent observation scores for all rats in the same group (n = 5); and the mean value for the vehicle group was 1.

The experimental procedure is as shown in FIG. 20 below.

After being left to stand at room temperature for 2 h, the blood was centrifuged at 4 °C at 4500 g for 10 min; the urine was centrifuged at 4 °C at 3220 g for 5 min; and the serum phosphorus concentration and urinary phosphorus concentration (phosphomolybdic acid ultraviolet endpoint colorimetric method), as well as urinary sodium concentration (indirect ion electrode method) were measured.

The results were expressed as mean ± standard error (Means±SEM), n = 5 animals/group. The urinary phosphorus excretion amount (or urinary sodium excretion amount) in the rats was normalized and corrected against the respective dietary phosphorus (or sodium) intake. The formula was as follows: normalized correction value of urinary phosphorus excretion amount (expressed as nP) = urinary phosphorus excretion amount ÷ dietary phosphorus intake; normalized correction value of urinary sodium excretion amount (expressed as nNa) = urinary sodium excretion amount ÷ dietary sodium intake; two-way analysis of variance was adopted; and fecal form score was verified by the non-parametric test. *,$p < 0.05$; **,$p < 0.01$; ***,$p < 0.001$; ****,$p < 0.0001$.

Experimental results

Serum phosphorus concentration

Compared with the serum phosphorus concentration of the rats in the vehicle control group, the serum phosphorus concentration of the rats was significantly reduced after 4 days of treatment with the compound represented by formula (I) at the dose of 0.5 mg/kg or 1.0 mg/kg; after 10 days of treatment at the doses of 0.01 mg/kg, 0.05 mg/kg, 0.5 mg/kg, and 1.0 mg/kg, the serum phosphorus concentration of the rats was significantly reduced; and except for the highest dose group of 1.0 mg/kg, serum phosphorus tended to be stable in other groups. The effect of the compound represented by formula (I) on reducing serum phosphorus concentration at the dose of 0.01 mg/kg was similar to that of Tenapanor at the dose of 0.05 mg/kg. See FIG. 21 below for details.

Urinary phosphorus excretion amount

The 24-h nP of the rats in each group was compared with that of the rats in the vehicle-treated group. After 1 day of

treatment with the compound represented by formula (I) at the doses of 0.01 mg/kg, 0.05 mg/kg, 0.5 mg/kg, and 1.0 mg/kg, the nP of the rats showed a decreasing trend. The higher the dose, the lower the nP, and the nP was significantly reduced at the dose of 1.0 mg/kg. After 10 days of treatment, the nP was all significantly reduced at the doses of 0.01 mg/kg, 0.05 mg/kg, 0.5 mg/kg, and 1.0 mg/kg. After 15 days, the nP of the rats tended to be stable, but the nP of the rats in the low-dose group (0.01 mg/kg) showed a rising trend. The effect of the compound represented by formula (I) at the dose of 0.01 mg/kg on reducing nP in the rats was similar to that of Tenapanor at the dose of 0.05 mg/kg. See FIG. 22 below for details.

Urinary sodium excretion amount

The 24-h nNa of the rats in each group was compared with that of the rats in the vehicle control group. After 1 day of treatment with the compound represented by formula (I) at the doses of 0.01 mg/kg, 0.05 mg/kg, 0.5 mg/kg, and 1.0 mg/kg, the nNa of the rats was significantly reduced. However, after 10 days of treatment, the nNa of the rats showed a rising trend at the doses of 0.01 mg/kg and 0.05 mg/kg. See FIG. 23 below for details.

Fecal form score

Compared with the rats in the vehicle control group, the fecal form score of the rats was increased significantly after 1 day of treatment with the compound represented by formula (I) at the doses of 0.05 mg/kg, 0.5 mg/kg, and 1.0 mg/kg. The rats in the high-dose groups of 0.5 mg/kg and 1.0 mg/kg had more severe loose feces, but as the administration continued, the frequency of loose feces observed decreased. No rats had loose feces after the treatment at a low dose of 0.01 mg/kg. See FIG. 24 for details.

In the specification, description involving the term "one embodiment", "some embodiments", "examples", "a specific example", "some examples", or the like means that a particular feature, structure, material, or characteristic described in reference to the embodiment or example is included in at least one embodiment or example of the present disclosure. In this specification, the schematic descriptions of the terms described above do not necessarily refer to the same embodiment or example. Moreover, the specific features, materials, structures, and other characteristics described may be combined in any one or more embodiments or examples in an appropriate manner. Moreover, various embodiments or examples and features of various embodiments or examples described in this specification can be combined by those skilled in the art to the extent that they do not contradict each other.

Although examples of the present disclosure are illustrated and described above, it will be appreciated that the above examples are exemplary and not to be construed as limiting the present disclosure, and that changes, modifications, substitutions, and alterations can be made to the above examples by those of ordinary skill in the art within the scope of the present disclosure.

## Claims

1. An amorphous form of a compound represented by formula (I),

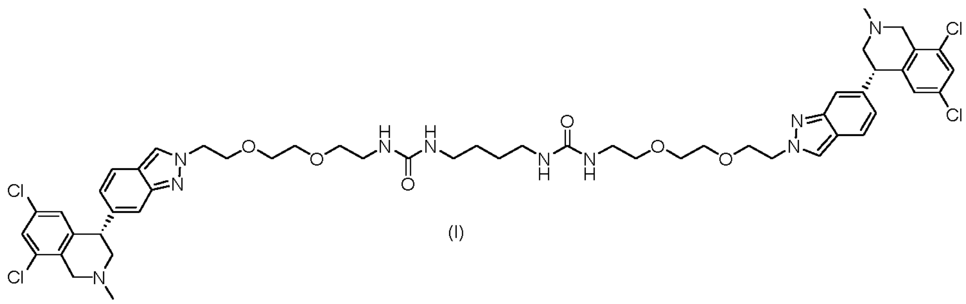

(I)

.

2. A 1,5-naphthalenedisulfonate of a compound represented by formula (I), having a structure as shown below:

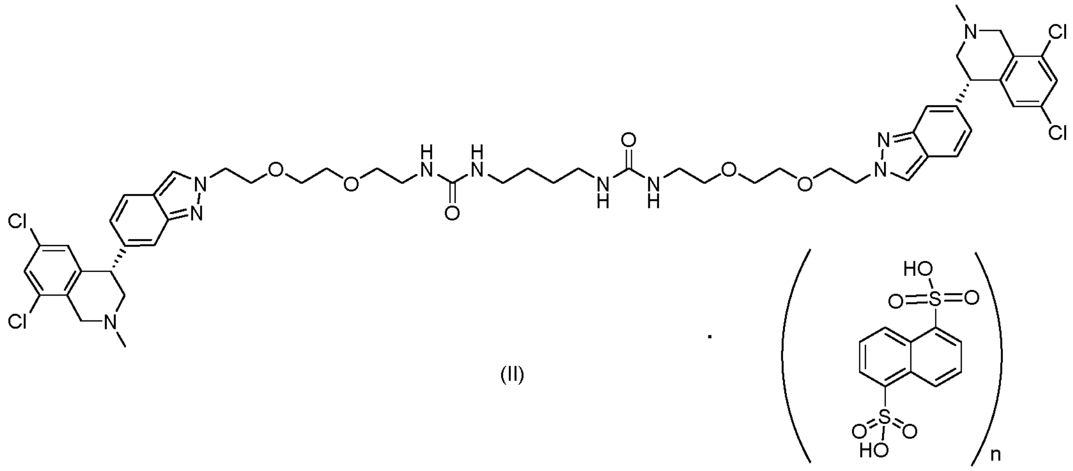

(II)

,

wherein

n = 0.9-2.0.

**3.** A method for preparing a 1,5-naphthalenedisulfonate of a compound represented by formula (I), comprising a reaction as shown below,

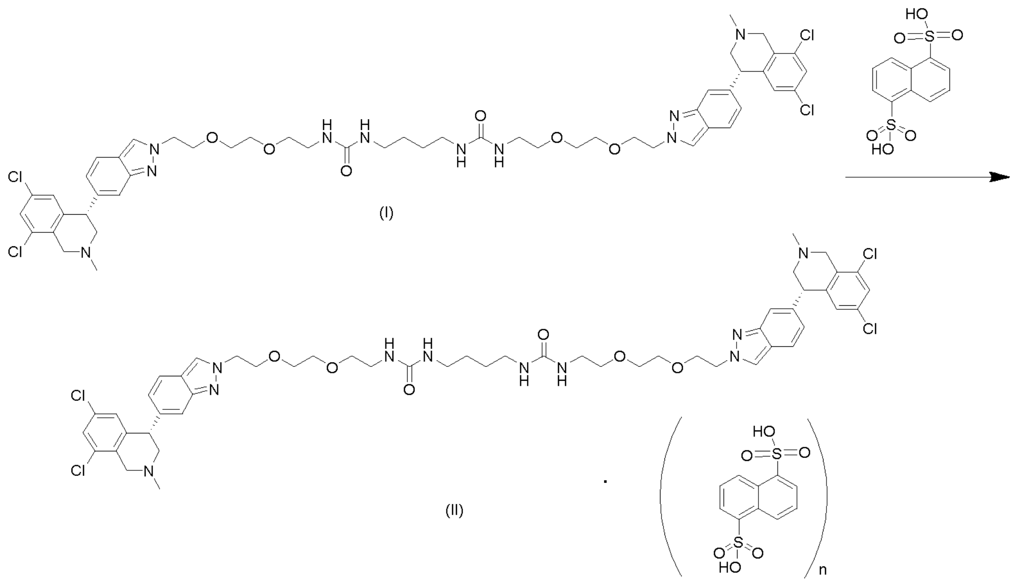

(I)

(II)

wherein

n = 0.9-2.0; and
a reaction solvent is selected from isopropanol, ethyl acetate, or a mixture of isopropanol and ethyl acetate.

**4.** The method according to claim 3, wherein the feeding molar ratio of the compound represented by formula (I) to 1,5-naphthalenedisulfonic acid is 1:(1-4);

optionally, the molar volume ratio of the compound represented by formula (I) to isopropanol is (5-15) mmol:(50-150) mL;
optionally, the molar volume ratio of the compound represented by formula (I) to ethyl acetate is (5-15) mmol:(50-150) mL;
optionally, the method further comprises a stirring treatment, a suction filtration treatment, and a drying treatment after the reaction is completed;

optionally, the stirring treatment is performed at room temperature for two days;
optionally, the suction filtration treatment is performed under nitrogen atmosphere;
optionally, the drying treatment is performed under a vacuum condition at room temperature for 2 h.

**5.** A hydrochloride of a compound represented by formula (I), having a structure as shown below:

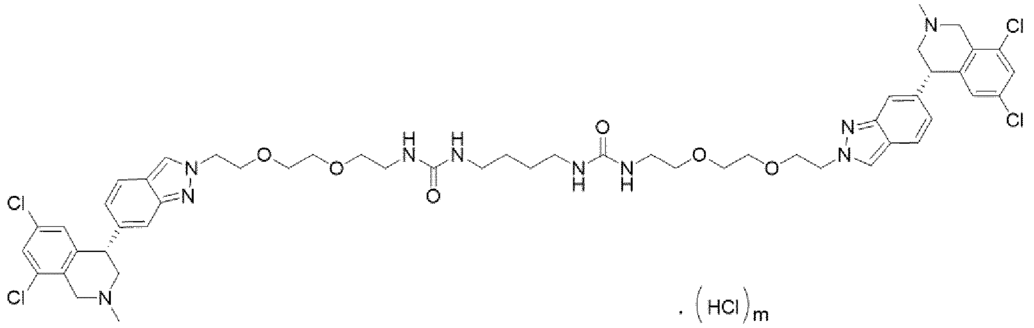

(III)

,

wherein

m = 1.6-4.2.

**6.** A method for preparing a compound represented by formula (III-1), comprising a reaction as shown below,

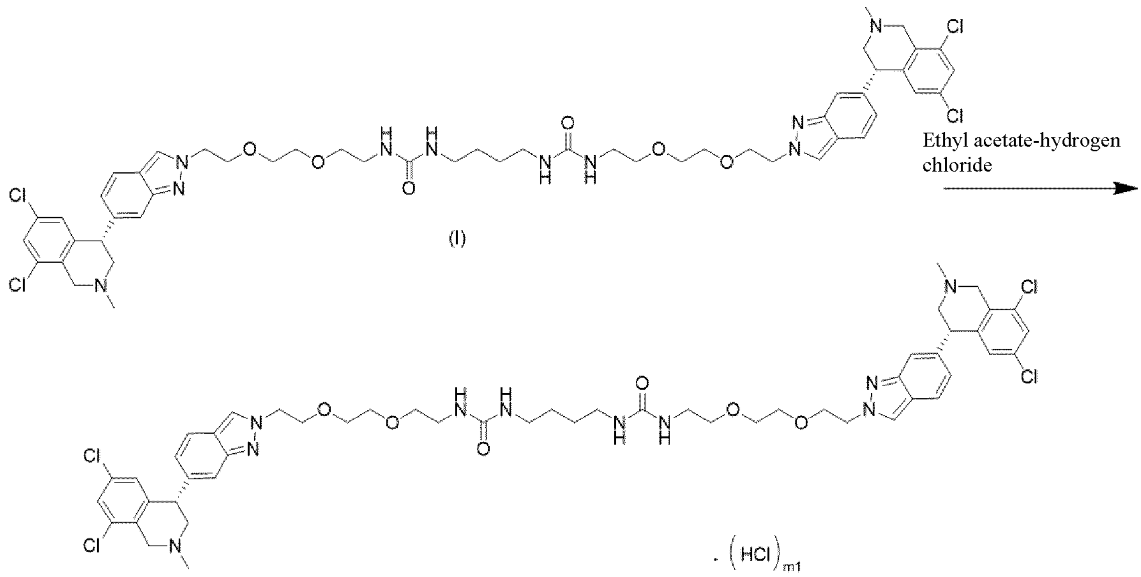


(I)

(III-1)

,

wherein

ml = 1.6-2.5; and
a reaction solvent is selected from methyl *tert-butyl* ether.

**7.** The method according to claim 6, wherein the feeding molar ratio of the compound represented by formula (I) to ethyl acetate-hydrogen chloride is (2-10):(1-5);

optionally, the molar volume ratio of the compound represented by formula (I) to methyl *tert-butyl* ether is (5-15) mmol: (50-150) mL;
optionally, the method further comprises a stirring treatment, a suction filtration treatment, and a drying treatment after the reaction;

optionally, the stirring treatment is performed at room temperature for two days;
optionally, the suction filtration treatment is performed under nitrogen atmosphere;
optionally, the drying treatment is performed under a vacuum condition at room temperature for 2 h.

**8.** A method for preparing a compound represented by formula (III-2), wherein

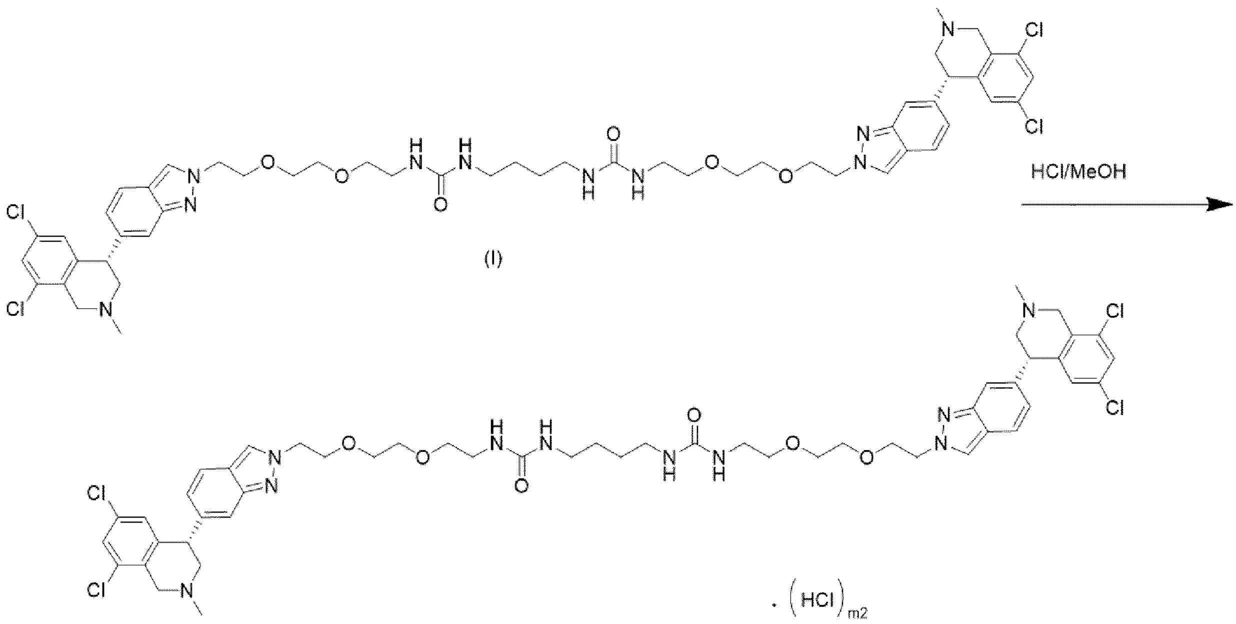


(I)

(III-2)

,

wherein

m2 = 3.8-4.2; and
a reaction solvent is methanol.

**9.** The method according to claim 8, wherein the reaction is performed under nitrogen atmosphere;

optionally, the feeding molar ratio of the compound represented by formula (I) to HCl/MeOH is (1-5):(2-10);
optionally, the molar volume ratio of the compound represented by formula (I) to methanol is (1-5) kg:(10-50) L;
optionally, a stirring treatment is performed for 30 min;
optionally, a slurring treatment is performed in ethyl acetate for 2 h.

**10.** Use of the amorphous form of the compound represented by formula (I) according to claim 1, or the 1,5-naphthalenedisulfonate of the compound represented by formula (I) according to claim 2, or the 1,5-naphthalenedisulfonate of the compound represented by formula (I) prepared by the method according to claim 3 or 4, or the hydrochloride of the compound represented by formula (I) according to claim 5, or the compound represented by formula (III-1) prepared by the method according to claim 6 or 7, or the compound represented by formula (III-2) prepared by the method according to claim 8 or 9 for a medicament for the inhibition of NHE-mediated antiport of a sodium ion or a hydrogen ion.

**11.** Use of the amorphous form of the compound represented by formula (I) according to claim 1, or the 1,5-naphthalenedisulfonate of the compound represented by formula (I) according to claim 2, or the 1,5-naphthalenedisulfonate of the compound represented by formula (I) prepared by the method according to claim 3 or 4, or the hydrochloride of the compound represented by formula (I) according to claim 5, or the compound represented by formula (III-1) prepared by the method according to claim 6 or 7, or the compound represented by formula (III-2) prepared by the method according to claim 8 or 9 for manufacturing a medicament for the treatment of a disease selected from irritable bowel syndrome, heart failure, chronic kidney disease, end-stage renal disease, and liver disease.

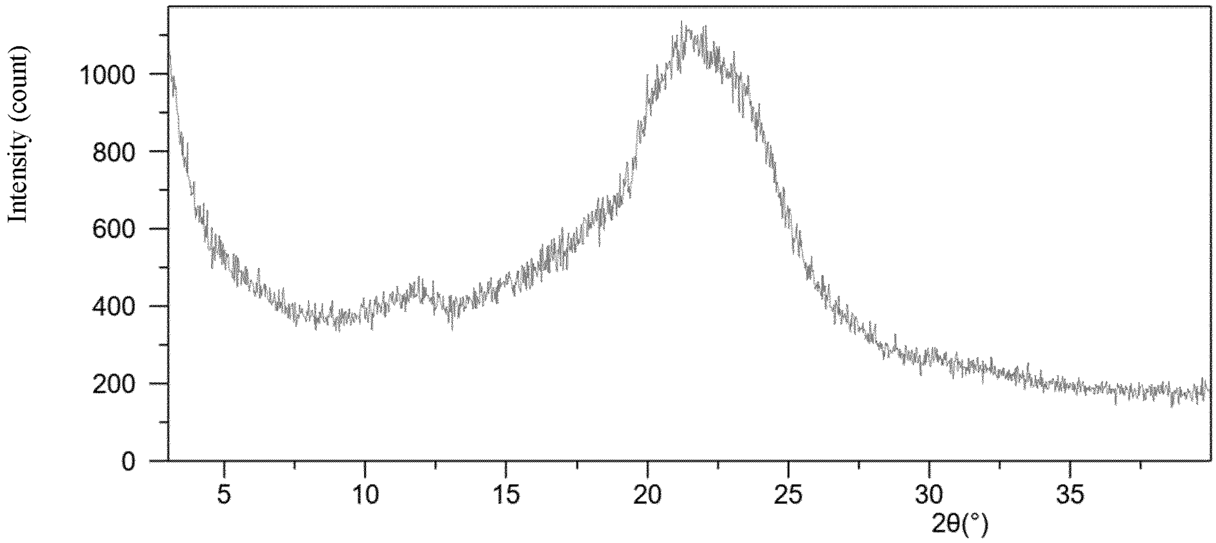
Intensity (count)
1000
800
600
400
200
0
5
10
15
20
25
30
35
2θ(°)

FIG. 1

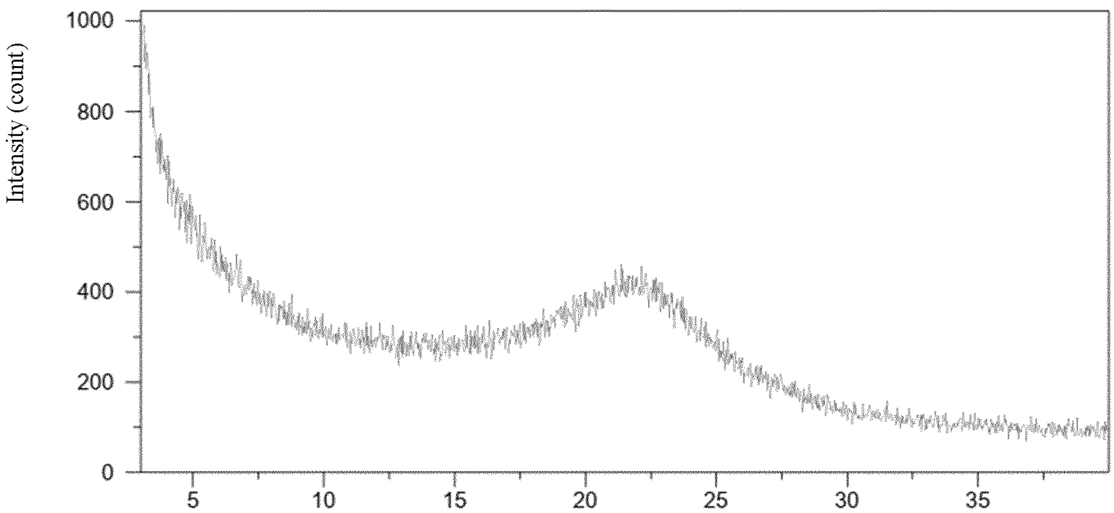
Intensity (count)
1000
800
600
400
200
0
5
10
15
20
25
30
35
2 θ(°)

FIG. 2

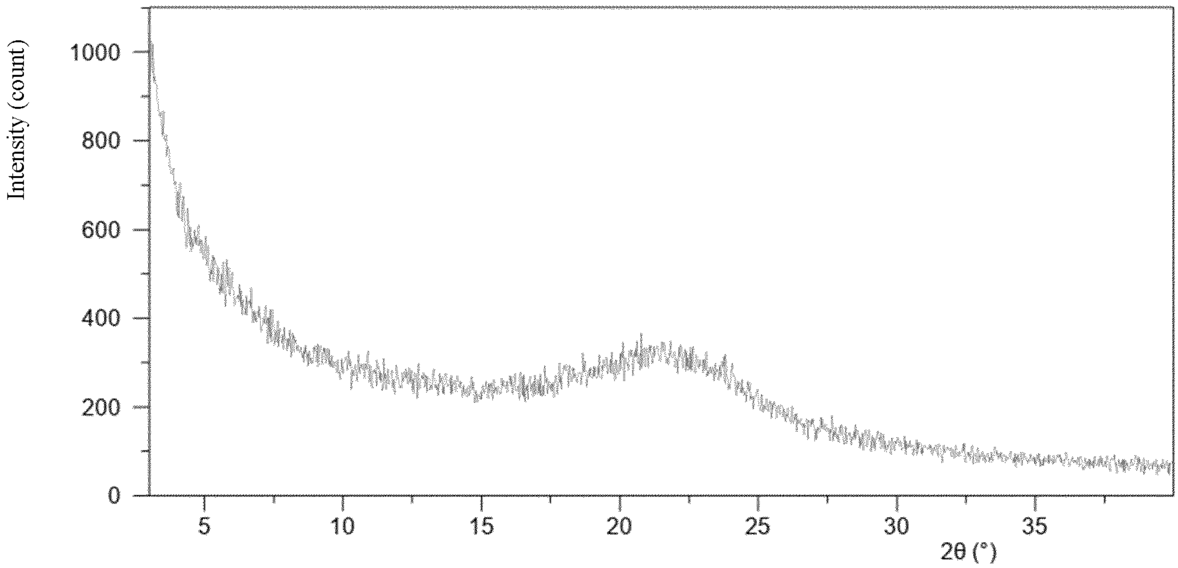
Intensity (count)
1000
800
600
400
200
0
5
10
15
20
25
30
35
2θ (°)

FIG. 3

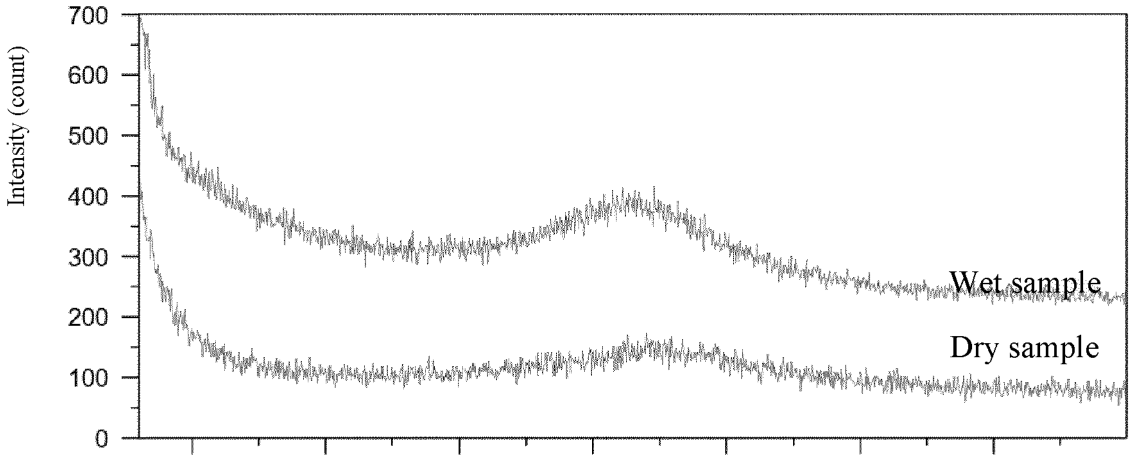
Intensity (count)
700
600
500
400
300
200
100
0
5
10
15
20
25
30
35
Wet sample
Dry sample

FIG. 4

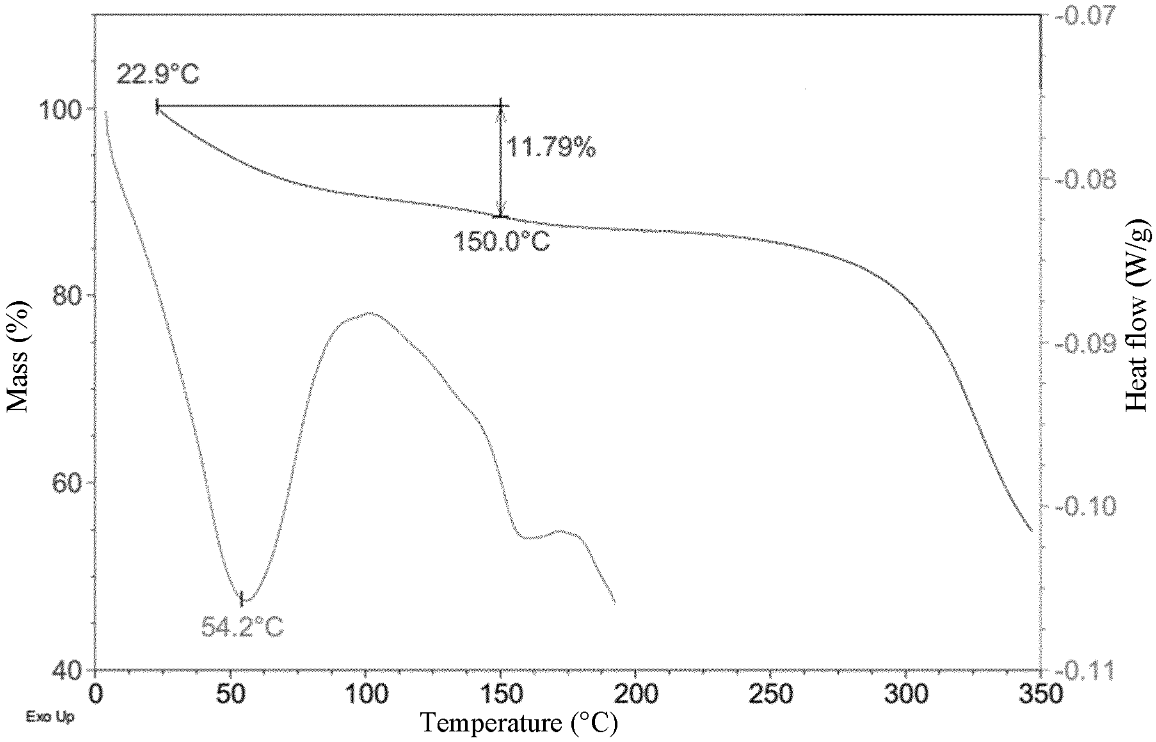
22.9°C
11.79%
150.0°C
54.2°C
Mass (%)
Heat flow (W/g)
Temperature (°C)
Exo Up
100
80
60
40
0
50
100
150
200
250
300
350
-0.07
-0.08
-0.09
-0.10
-0.11

FIG. 5

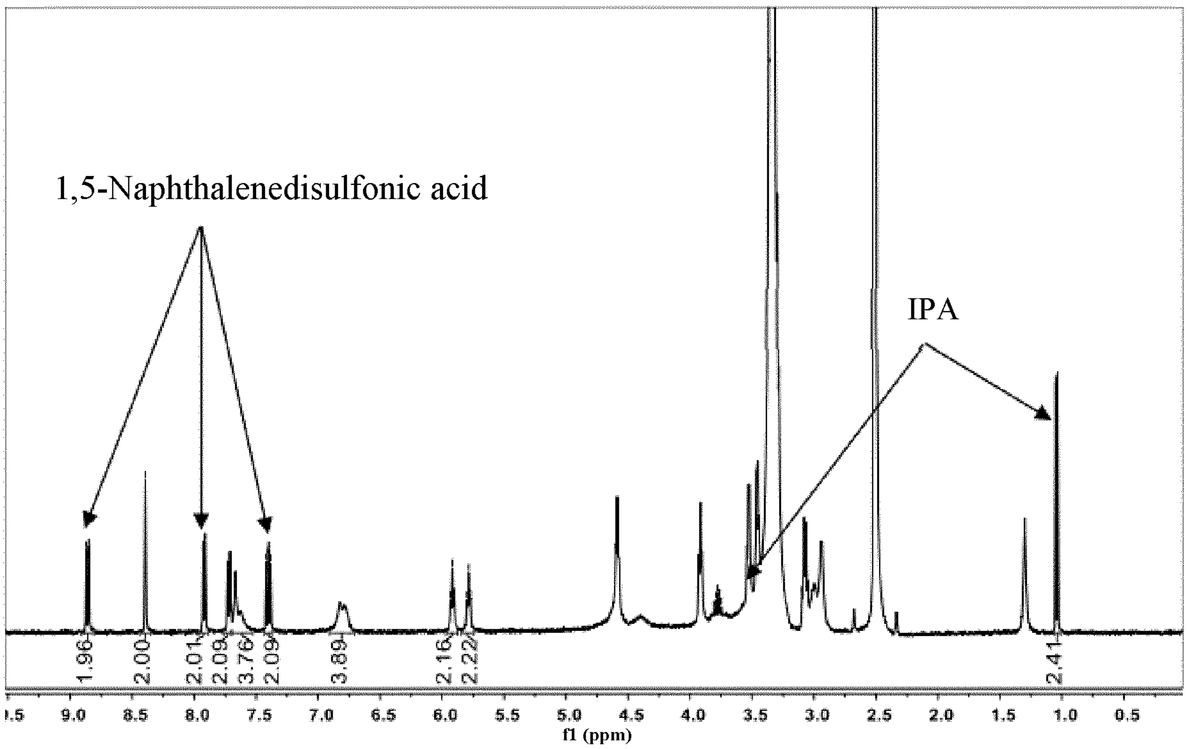
1,5-Naphthalenedisulfonic acid
IPA
1.96
2.00
2.01
2.09
3.76
2.09
3.89
2.16
2.22
2.41
f1 (ppm)

FIG. 6

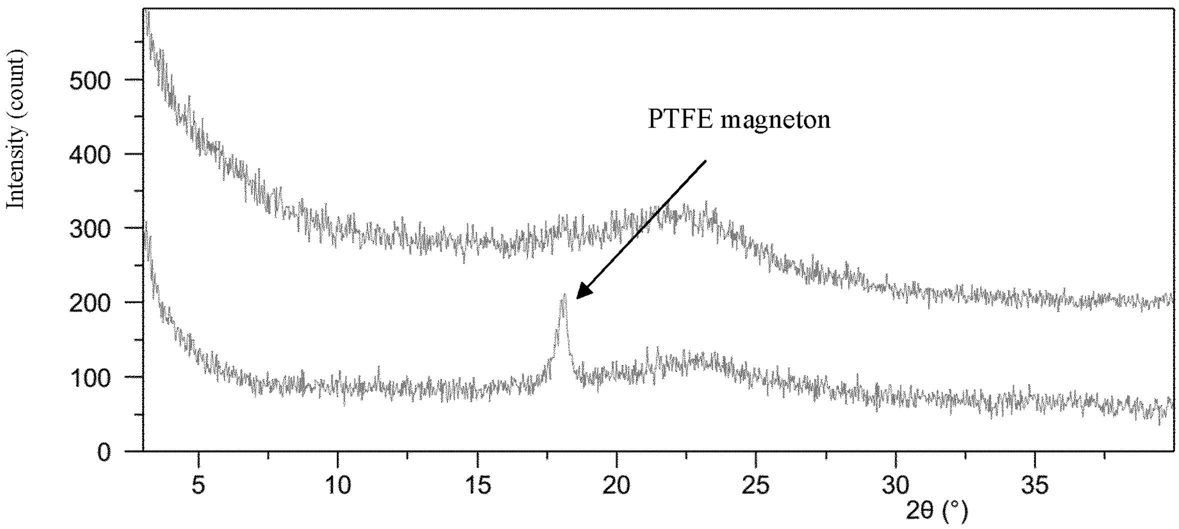

Intensity (count)
500
400
300
200
100
0
PTFE magneton
5
10
15
20
25
30
35
2θ (°)


FIG. 7

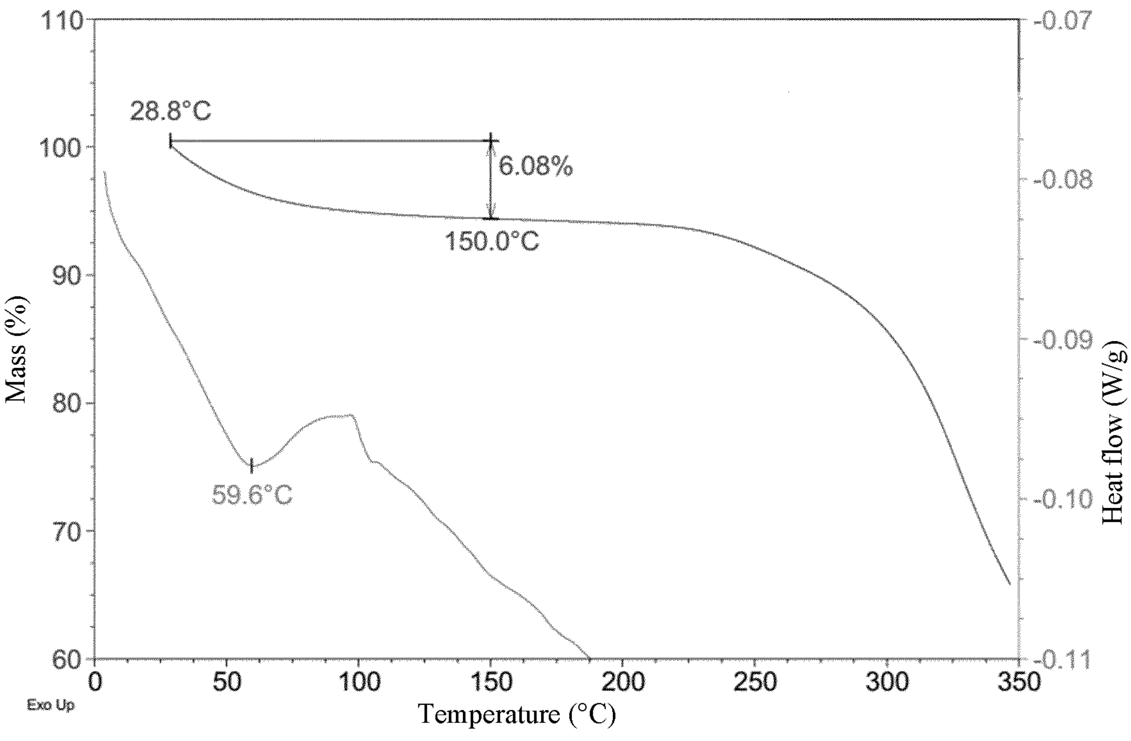

110
100
90
80
70
60
Mass (%)
28.8°C
6.08%
150.0°C
59.6°C
-0.07
-0.08
-0.09
-0.10
-0.11
Heat flow (W/g)
0
50
100
150
200
250
300
350
Exo Up
Temperature (°C)


FIG. 8

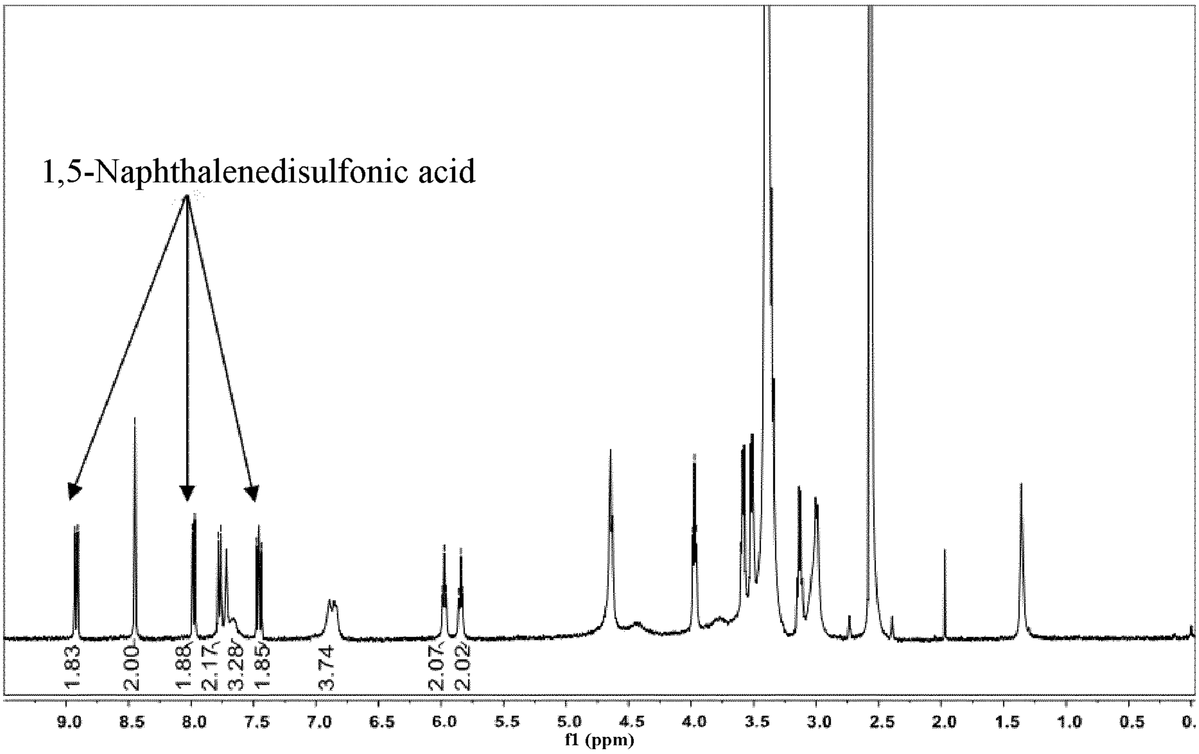
1,5-Naphthalenedisulfonic acid
1.83
2.00
1.88
2.17
3.28
1.85
3.74
2.07
2.02
9.0 8.5 8.0 7.5 7.0 6.5 6.0 5.5 5.0 4.5 4.0 3.5 3.0 2.5 2.0 1.5 1.0 0.5 0.
f1 (ppm)

FIG. 9

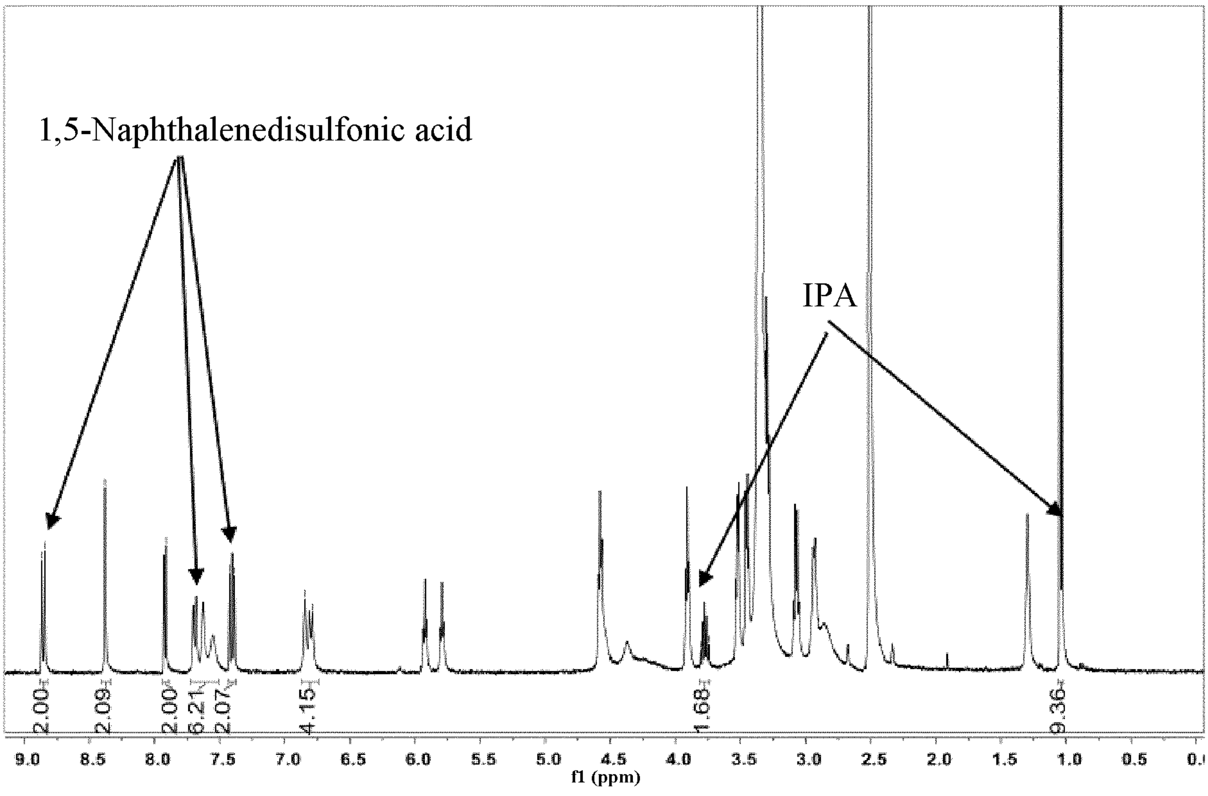
1,5-Naphthalenedisulfonic acid
IPA
2.00
2.09
2.00
6.21
2.07
4.15
1.68
9.36
9.0 8.5 8.0 7.5 7.0 6.5 6.0 5.5 5.0 4.5 4.0 3.5 3.0 2.5 2.0 1.5 1.0 0.5 0.
f1 (ppm)

FIG. 10

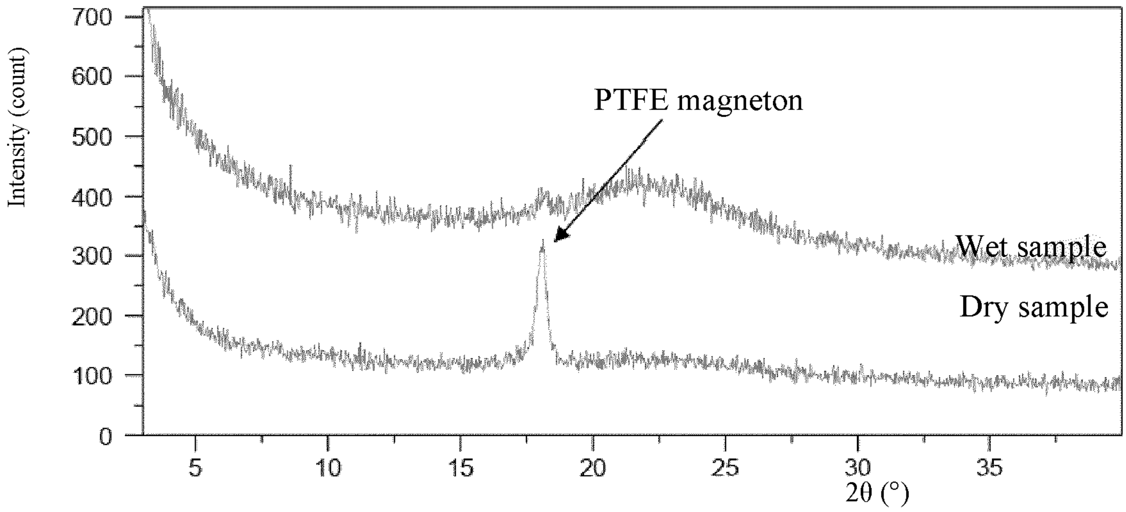

Intensity (count)
700
600
500
400
300
200
100
0
PTFE magneton
Wet sample
Dry sample
5
10
15
20
25
30
35
2θ (°)


FIG. 11

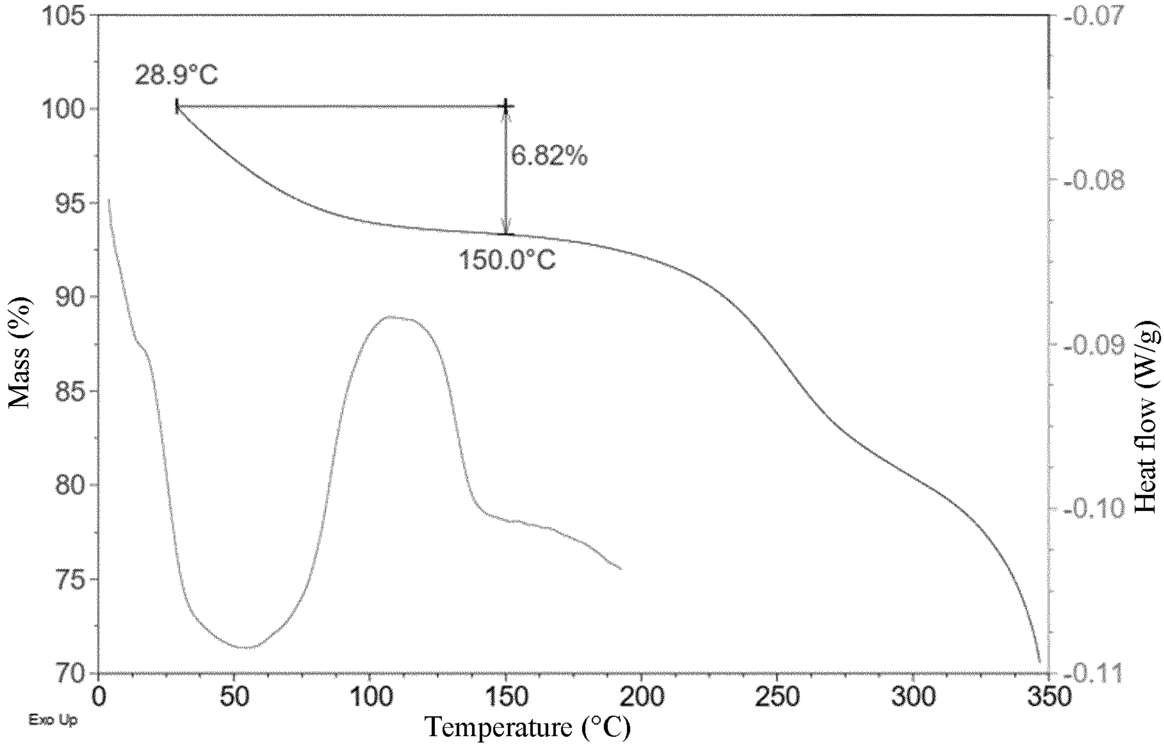

105
100
95
90
85
80
75
70
Mass (%)
28.9°C
6.82%
150.0°C
-0.07
-0.08
-0.09
-0.10
-0.11
Heat flow (W/g)
0
50
100
150
200
250
300
350
Exo Up
Temperature (°C)


FIG. 12

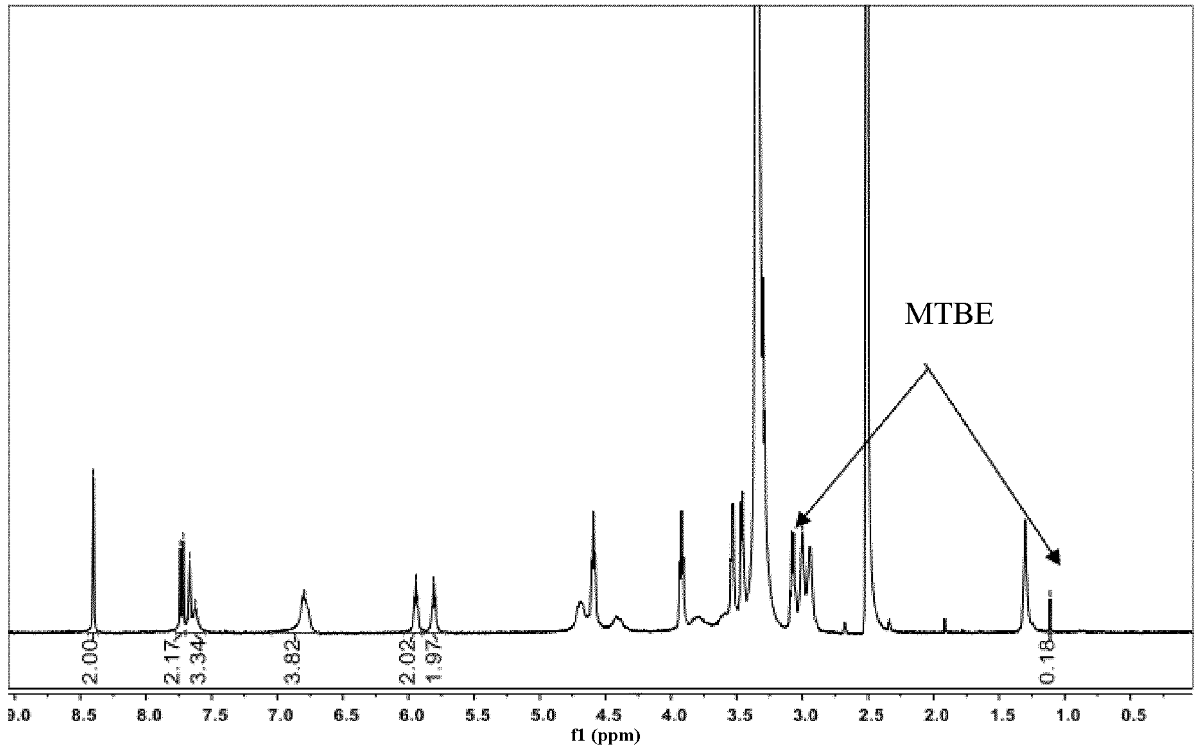
MTBE
2.00
2.17
3.34
3.82
2.02
1.97
0.18
9.0 8.5 8.0 7.5 7.0 6.5 6.0 5.5 5.0 4.5 4.0 3.5 3.0 2.5 2.0 1.5 1.0 0.5
f1 (ppm)

FIG. 13

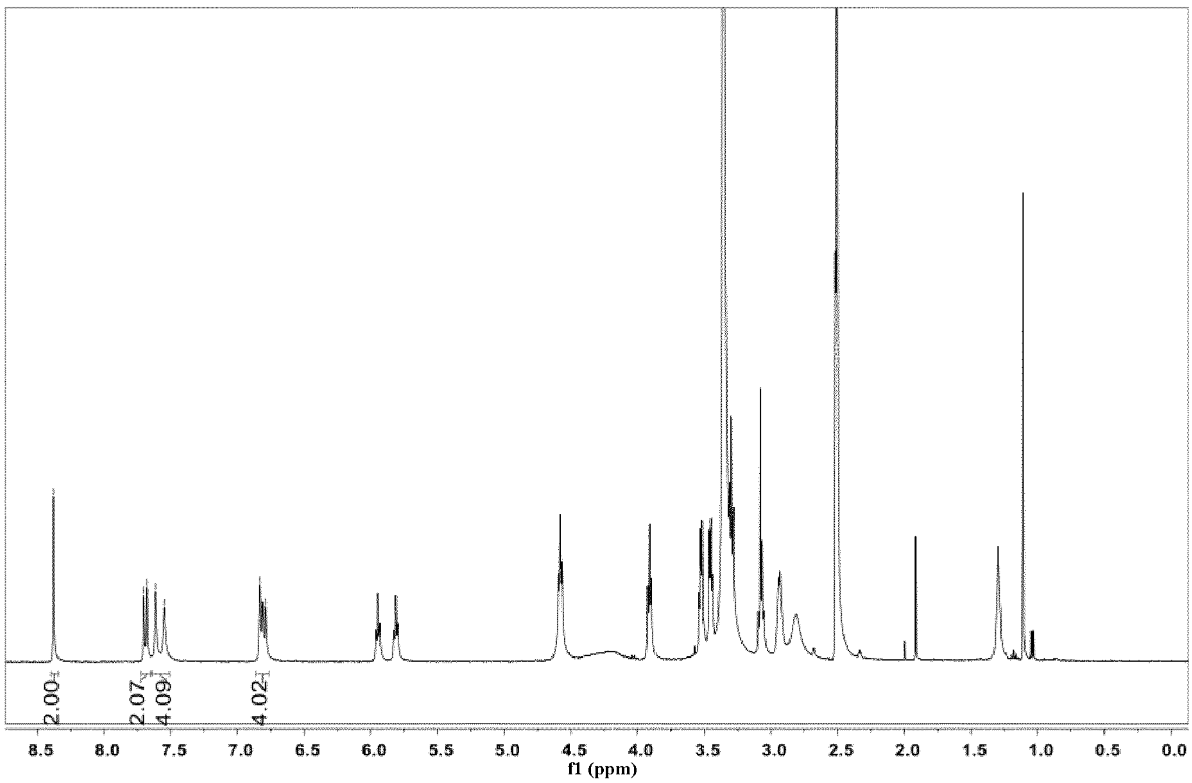
2.00
2.07
4.09
4.02
8.5 8.0 7.5 7.0 6.5 6.0 5.5 5.0 4.5 4.0 3.5 3.0 2.5 2.0 1.5 1.0 0.5 0.0
f1 (ppm)

FIG. 14

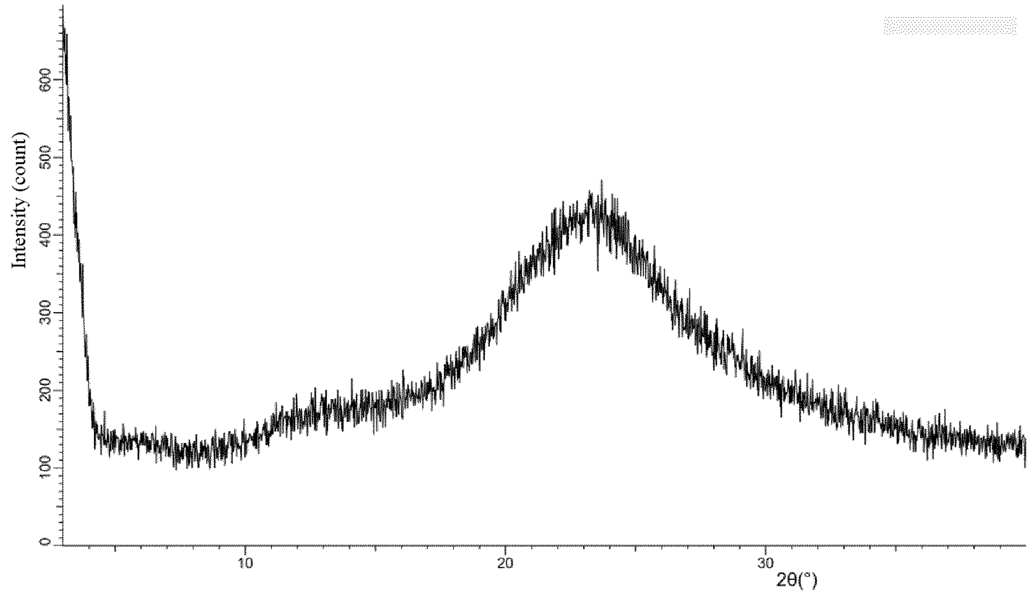
Intensity (count)
0
100
200
300
400
500
600
10
20
30
2θ(°)

FIG. 15

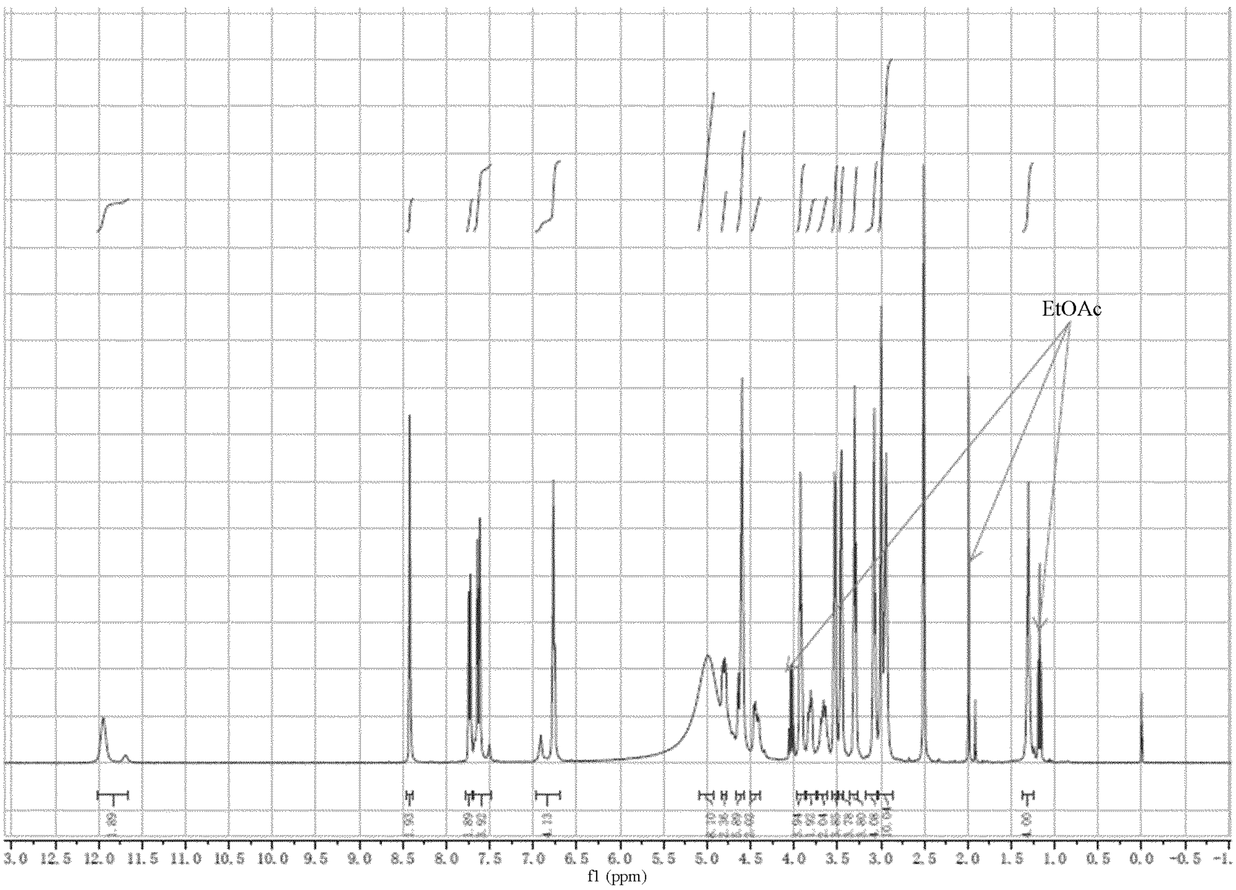
EtOAc
f1 (ppm)

FIG. 16

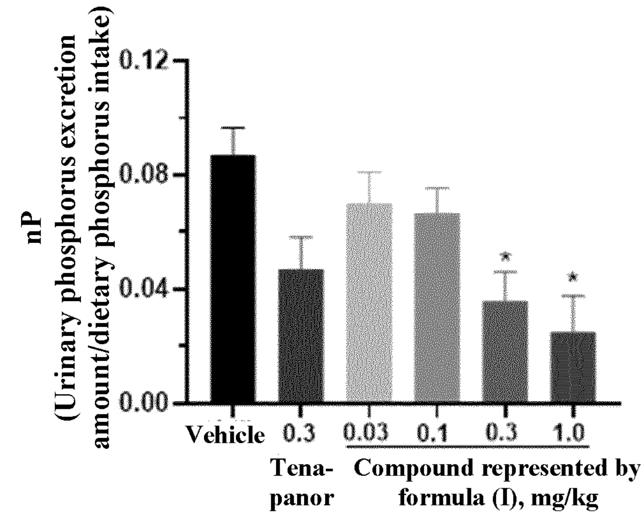


FIG. 17

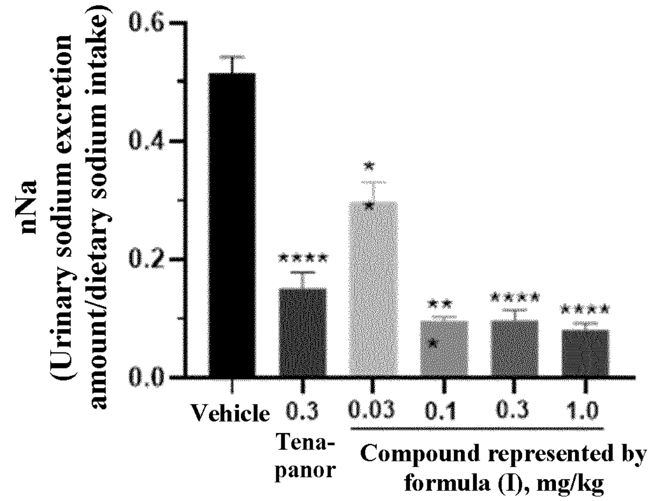


FIG. 18

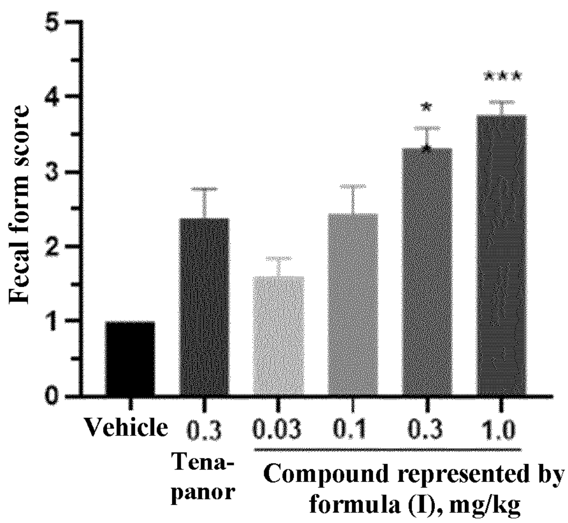


FIG. 19

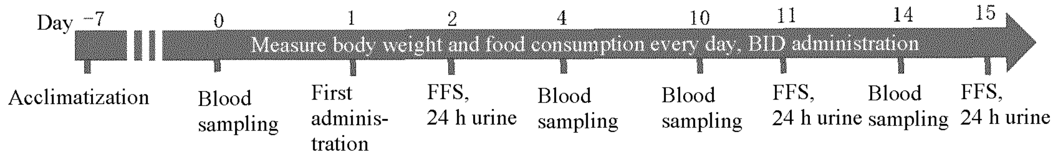


FIG. 20

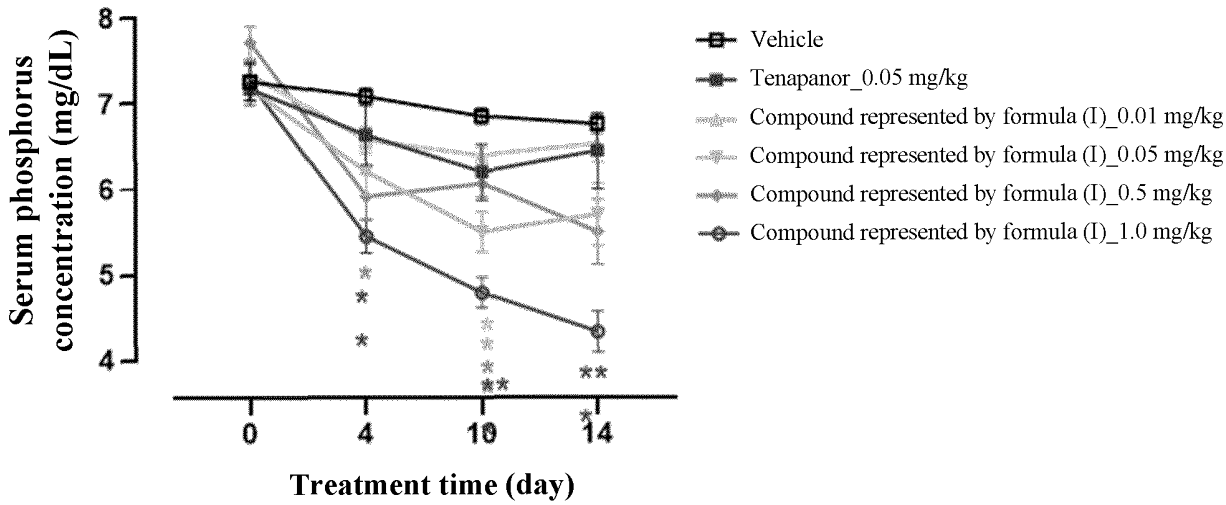


FIG. 21

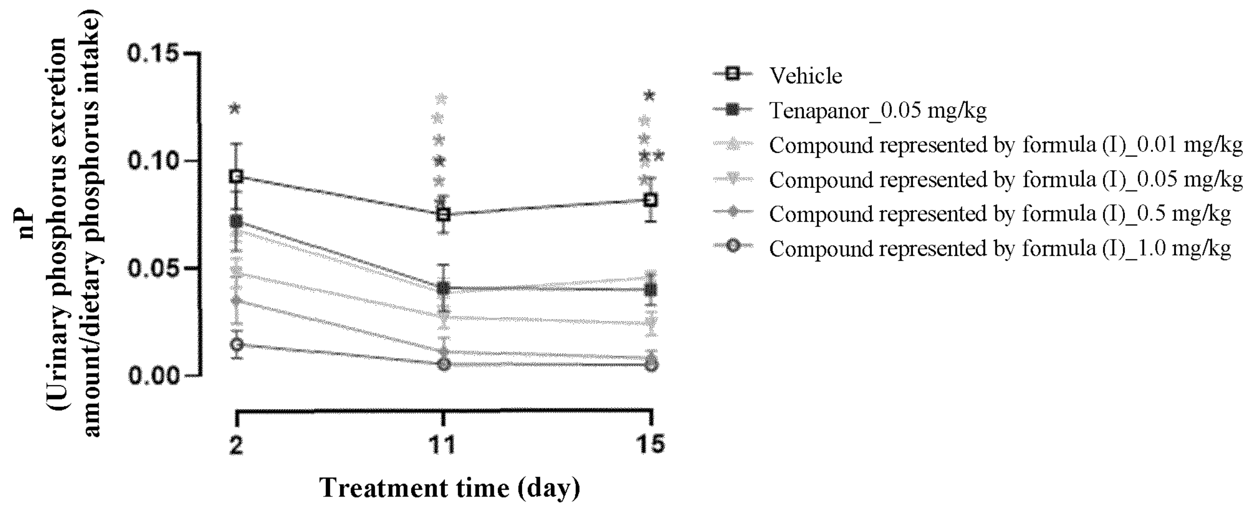


FIG. 22

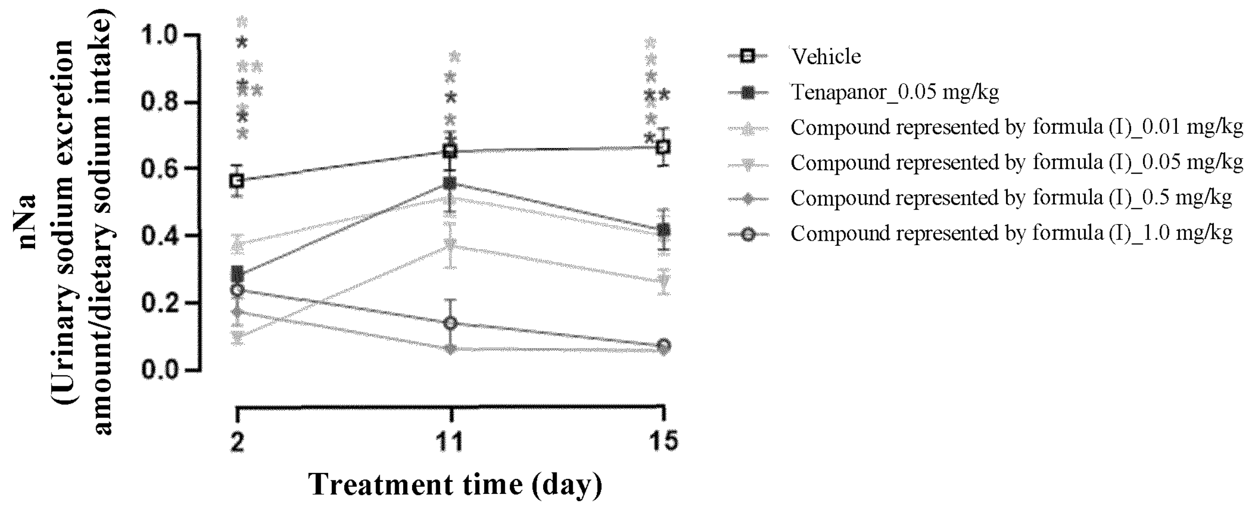
nNa
(Urinary sodium excretion amount/dietary sodium intake)
1.0
0.8
0.6
0.4
0.2
0.0
2
11
15
Treatment time (day)
Vehicle
Tenapanor_0.05 mg/kg
Compound represented by formula (I)_0.01 mg/kg
Compound represented by formula (I)_0.05 mg/kg
Compound represented by formula (I)_0.5 mg/kg
Compound represented by formula (I)_1.0 mg/kg

FIG. 23

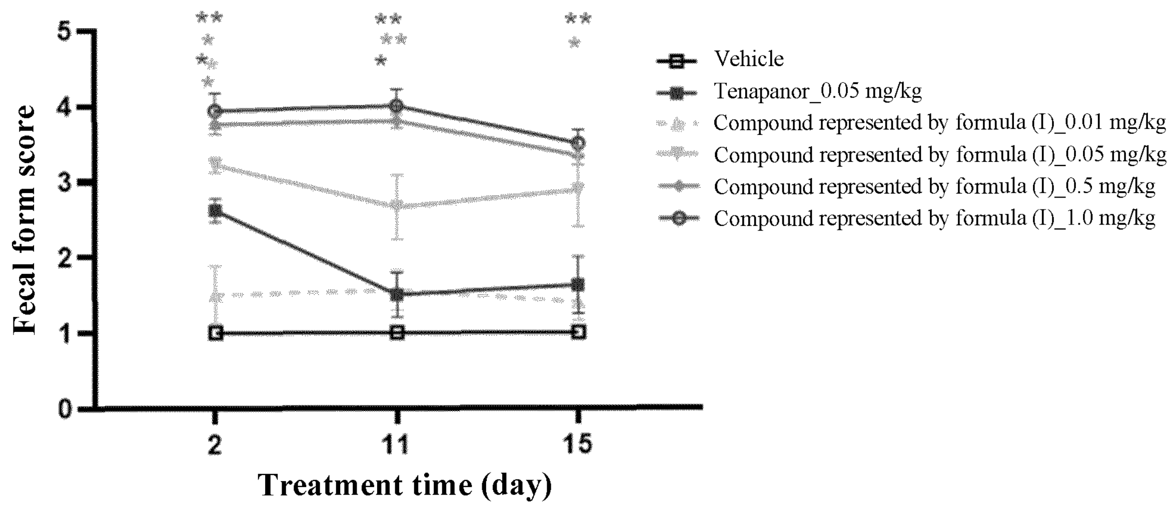
Fecal form score
5
4
3
2
1
0
2
11
15
Treatment time (day)
Vehicle
Tenapanor_0.05 mg/kg
Compound represented by formula (I)_0.01 mg/kg
Compound represented by formula (I)_0.05 mg/kg
Compound represented by formula (I)_0.5 mg/kg
Compound represented by formula (I)_1.0 mg/kg

FIG. 24

## INTERNATIONAL SEARCH REPORT

International application No.
**PCT/CN2023/101233**

**A. CLASSIFICATION OF SUBJECT MATTER**

C07D401/14(2006.01)i; A61P1/00(2006.01)i; A61P37/08(2006.01)i; A61P9/04(2006.01)i; A61P13/12(2006.01)i; A61P1/16(2006.01)i

According to International Patent Classification (IPC) or to both national classification and IPC

**B. FIELDS SEARCHED**

Minimum documentation searched (classification system followed by classification symbols)

IPC:C07D,A61P

Documentation searched other than minimum documentation to the extent that such documents are included in the fields searched

Electronic data base consulted during the international search (name of data base and, where practicable, search terms used)

CNKI; VEN; CNTXT; CNABS; WOTXT; STN CAPLUS; REGISTRY; EPTXT; USTXT; 万方, WANFANG: 上海济煜, 苯并杂环, 四氢异喹啉, NHE, 抑制剂, 钠离子, 氢离子, 反向转运, 肾, 心力衰竭, inhibitor, benzoheterocycle, tetrahydroisoquinoline, sodium ion, hudrogen ion, reverse transport, kidney, heart failure

**C. DOCUMENTS CONSIDERED TO BE RELEVANT**

| Category* | Citation of document, with indication, where appropriate, of the relevant passages | Relevant to claim No. |
|---|---|---|
| PX | CN 114702478 A (SHANGHAI JEMINCARE PHARMACEUTICAL CO., LTD. et al.) 05 July 2022 (2022-07-05)<br>claims 17-19, description, paragraph 123 | 1-9, 11 |
| A | CN 102333759 A (ARDELYX, INC.) 25 January 2012 (2012-01-25)<br>entire document | 1-9, 11 |
| A | US 2017340623 A1 (ARDELYX, INC.) 30 November 2017 (2017-11-30)<br>entire document | 1-9, 11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 October 2023** | **23 October 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/101233**

**Box No. II** **Observations where certain claims were found unsearchable (Continuation of item 2 of first sheet)**

This international search report has not been established in respect of certain claims under Article 17(2)(a) for the following reasons:

1. ☑ Claims Nos.: **10**
because they relate to subject matter not required to be searched by this Authority, namely:

Claim 10 relates to a treatment method implemented on the human/animal body. Therefore, the subject matter of claim 10 falls within subject matter for which no search is required by the International Searching Authority. (PCT Rule 39.1(iv))

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/101233**

| Patent document cited in search report | Publication date (day/month/year) | Patent family member(s) | Publication date (day/month/year) |
|---|---|---|---|
| CN 114702478 A | 05 July 2022 | IL 303799 A | 01 August 2023 |
| | | AU 2021401102 A1 | 03 August 2023 |
| | | CA 3202573 A1 | 23 June 2022 |
| | | WO 2022127917 A1 | 23 June 2022 |
| | | TW 202233599 A | 01 September 2022 |
| | | TWI 805137 B | 11 June 2023 |
| CN 102333759 A | 25 January 2012 | JP 2012514009 A | 21 June 2012 |
| | | JP 5502106 B2 | 28 May 2014 |
| | | IL 213852 A0 | 31 July 2011 |
| | | IL 213852 A | 28 February 2017 |
| | | DK 2384318 T3 | 19 February 2018 |
| | | PL 2384318 T3 | 30 April 2018 |
| | | KR 20170091783 A | 09 August 2017 |
| | | EP 2384318 A2 | 09 November 2011 |
| | | EP 2384318 B1 | 15 November 2017 |
| | | ES 2657938 T3 | 07 March 2018 |
| | | US 2013274285 A1 | 17 October 2013 |
| | | US 9006281 B2 | 14 April 2015 |
| | | CA 2748607 A1 | 08 July 2010 |
| | | MX 2011007024 A | 27 September 2011 |
| | | HUE 036405 T2 | 30 July 2018 |
| | | US 2012263670 A1 | 18 October 2012 |
| | | US 8541448 B2 | 24 September 2013 |
| | | IL 259851 A | 31 July 2018 |
| | | IL 259851 B | 30 July 2020 |
| | | US 2015190389 A1 | 09 July 2015 |
| | | US 9408840 B2 | 09 August 2016 |
| | | LT 2384318 T | 26 February 2018 |
| | | AU 2009334511 A1 | 21 July 2011 |
| | | AU 2009334511 B2 | 28 January 2016 |
| | | AU 2009334511 C1 | 18 August 2016 |
| | | PT 2384318 T | 07 February 2018 |
| | | US 2014107074 A1 | 17 April 2014 |
| | | US 8969377 B2 | 03 March 2015 |
| | | CY 1120451 T1 | 10 July 2019 |
| | | EP 3351248 A1 | 25 July 2018 |
| | | EP 3351248 B1 | 09 June 2021 |
| | | KR 20220042487 A | 05 April 2022 |
| | | EP 3939964 A1 | 19 January 2022 |
| | | HRP 20180289 T1 | 23 March 2018 |
| | | MX 345283 B | 24 January 2017 |
| | | KR 20110110287 A | 06 October 2011 |
| | | KR 101683318 B1 | 07 December 2016 |
| | | KR 20200111230 A | 28 September 2020 |
| | | NO 2384318 T3 | 14 April 2018 |
| | | SI 2384318 T1 | 30 March 2018 |
| | | IL 250641 A0 | 30 April 2017 |
| | | IL 250641 B | 28 June 2018 |
| | | JP 2014114300 A | 26 June 2014 |
| | | JP 5805802 B2 | 10 November 2015 |

Form PCT/ISA/210 (patent family annex) (July 2022)

**INTERNATIONAL SEARCH REPORT**
**Information on patent family members**

International application No.
**PCT/CN2023/101233**

| Patent document cited in search report | Publication date (day/month/year) | Patent family member(s) | Publication date (day/month/year) |
|---|---|---|---|
| | | HK 1198162 A1 | 13 March 2015 |
| | | WO 2010078449 A2 | 08 July 2010 |
| | | WO 2010078449 A3 | 30 September 2010 |
| | | KR 20160140994 A | 07 December 2016 |
| | | KR 101766619 B1 | 08 August 2017 |
| | | BRPI 0923861 A2 | 28 July 2015 |
| | | BRPI 0923861 B1 | 10 March 2020 |
| | | BRPI 0923861 B8 | 25 May 2021 |
| US 2017340623 A1 | 30 November 2017 | US 2020268741 A1 | 27 August 2020 |
| | | US 11318129 B2 | 03 May 2022 |
| | | US 10543207 B2 | 28 January 2020 |
| | | US 2019374533 A1 | 12 December 2019 |
| | | US 2023031776 A1 | 02 February 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210701699
- CN 2021139314 W